# EUROPEAN PATENT SPECIFICATION

(11) **EP 2 401 294 B1**
(45) Date of publication and mention of the grant of the patent: **05.09.2018**
(21) Application number: 10706603.7
(22) Date of filing: 25.02.2010
(51) Int. Cl.: C07K 16/00, C12N 15/64, C07K 16/24

(54) **METHOD FOR PRODUCING ANTIBODIES**
VERFAHREN ZUR HERSTELLUNG VON ANTIKÖRPERN
PROCÉDÉ DE PRODUCTION D'ANTICORPS

(30) Priority: 25.02.2009 GB 0903210; 25.02.2009 GB 0903209
(43) Date of publication of application: 04.01.2012
(73) Proprietor: UCB Biopharma SPRL, 1070 Brussels (BE)
(72) Inventor: STEPHENS, Paul, Edward, Slough, Berkshire SL1 3WE (GB); WRIGHT, Michael, John, Slough, Berkshire SL1 3WE (GB)
(74) Representative: Thompson, John
(86) International application number: PCT/EP2010/052408
(87) International publication number: WO 2010/097435

(56) References cited:
- EP-A2- 1 516 929
- EP-B1- 2 401 295
- WO-A1-92/02551
- WO-A1-2004/051268
- WO-A1-2004/106377
- WO-A1-2005/019823
- WO-A1-2005/019824
- WO-A1-2005/121789
- WO-A2-2004/032841
- LIAO H X ET AL: "High-throughput isolation of immunoglobulin genes from single human B cells and expression as monoclonal antibodies" JOURNAL OF VIROLOGICAL METHODS, ELSEVIER BV, NL LNKD- DOI:10.1016/J.JVIROMET.2009.02.014, vol. 158, no. 1-2, 21 February 2009 (2009-02-21), pages 171-179, XP026022553 ISSN: 0166-0934 [retrieved on 2009-02-21]
- TILLER ET AL: "Efficient generation of monoclonal antibodies from single human B cells by single cell RT-PCR and expression vector cloning", JOURNAL OF IMMUNOLOGICAL METHODS, ELSEVIER SCIENCE PUBLISHERS B.V.,AMSTERDAM, NL, vol. 329, no. 1-2, 31 October 2007 (2007-10-31), pages 112-124, XP022389335, ISSN: 0022-1759
- BABCOOK J S ET AL: "A NOVEL STRATEGY FOR GENERATING MONOCLONAL ANTIBODIES FROM SINGLE, ISOLATED LYMPHOCYTES PRODUCING ANTIBODIES OF DEFINED SPECIFICITIES", PROCEEDINGS OF THE NATIONAL ACADEMY OF SCIENCES, NATIONAL ACADEMY OF SCIENCES, US, vol. 93, no. 15, 23 July 1996 (1996-07-23) , pages 7843-7848, XP000608647, ISSN: 0027-8424, DOI: 10.1073/PNAS.93.15.7843
- COLOMA M J ET AL: "Novel vectors for the expression of antibody molecules using variable regions generated by polymerase chain reaction", JOURNAL OF IMMUNOLOGICAL METHODS, ELSEVIER SCIENCE PUBLISHERS B.V.,AMSTERDAM, NL, vol. 152, no. 1, 31 July 1992 (1992-07-31) , pages 89-104, XP023657543, ISSN: 0022-1759, DOI: 10.1016/0022-1759(92)90092-8 [retrieved on 1992-07-31]
- MARLITT STECH ET AL: "Cell-Free Synthesis Meets Antibody Production: A Review", ANTIBODIES, vol. 4, no. 1, 26 January 2015 (2015-01-26), pages 12-33, XP055321918, DOI: 10.3390/antib4010012
- G. Kanter ET AL: "Cell-free production of scFv fusion proteins: an efficient approach for personalized lymphoma vaccines", Blood, vol. 109, no. 8, 15 April 2007 (2007-04-15), pages 3393-3399, XP055235253, US ISSN: 0006-4971, DOI: 10.1182/blood-2006-07-030593

## Description

The invention relates to a method for producing and expressing proteins with a desired function, particularly proteins which are components of a multimeric protein, such as an antibody. The present invention also relates to a polynucleotide suitable for expressing a fusion protein and a cell comprising the polynucleotide. The invention also relates to a host cell capable of expressing exogenous polynucleotides encoding a protein of interest.

Through the use of recombinant DNA, genes that are identified as important, for example in therapeutic applications, can be amplified and isolated. Cells are used extensively to produce a recombinant protein of interest by transfecting the cell with a vector comprising the polynucleotide sequence encoding the protein of interest. Cells may be used to produce any desired protein including multimeric proteins, such as antibodies. The use of mammalian cells for expressing recombinant proteins provides a "natural" protein expression pathway wherein the proteins are subjected to the cell's protein folding machinery and further downstream processing. This provides recombinant proteins having the required conformation for *in vivo* activity.

The continued advancement of mammalian transfection techniques is increasingly important for many applications. This is particularly the case when a large number of proteins of interest are to be expressed and analyzed, such as in high throughput antibody screening projects.

Many research, therapeutic and diagnostic proteins are fusion proteins comprising a polypeptide sequence encoded by two or more genes. Fusion proteins occur naturally and may also be produced by recombinant means. Fusion proteins may form multimeric proteins comprising a heterogeneous complex of a plurality of correctly folded polypeptide chains. The correct functionality of the multimeric protein depends on both the correct protein domain folding of each individual polypeptide chain and their correct association with each other. Antibodies are an example of such a multimeric protein comprising one or more fusion proteins. IgG antibodies comprise four separate polypeptide chains, two light chains and two heavy chains, wherein each chain is a fusion protein of the variable region and the constant region.

Currently the production of recombinant antibodies requires multiple digestion and ligation steps. Once the desired variable heavy and variable light chains have been identified, the encoding DNA of each chain must be produced and inserted into plasmid vectors. The plasmid vectors comprise the necessary transcription regulatory elements including promoters and terminators for each coding sequence. The vectors may also comprise polynucleotide sequences encoding the constant regions of the heavy and light chains. The vectors are then transfected into host cells. Once transfected into the host cells the vectors may employ the cell's transcription machinery to transcribe the encoding DNA sequence in the vectors, which is transient transfection. Alternatively, the encoding DNA sequence in the vectors may be integrated into the cell's genome, from which the DNA is transcribed, which is stable transfection. The light chain and heavy chain are then expressed by the host cells and various isolation and purification steps are carried out to obtain the final antibody product. The whole process to produce the final antibody product is extremely complex and time consuming.

This method is also necessary for the production of any recombinant multimeric protein comprising two or more component polypeptide chains which assemble to form the functional multimeric protein.

WO 2005/042774 discloses a multiplex overlap-extension RT-PCR method to link two or more nucleotide sequences encoding for domains or components of a heteromeric protein in a single reaction. This document discloses that the method may be used to link a cognate pair of non-contiguous nucleic acids of interest that are contained within or derived from a single cell. The method is disclosed as particularly useful for linking heavy chain variable region and light chain variable region encoding sequences from a single cell. The cognate pair may comprise one or more constant region encoding sequences in addition to the variable region encoding sequences. However, the resulting nucleotide product from the method still requires cloning and insertion into an appropriate expression vector to allow expression in a suitable host cell.

Liao et al Journal of Virological Methods vol 158, no. 1-2, 21 February 2009, pages 171-179 discloses high-throughput isolation of immunoglobulin genes from single human B cells and expression as monoclonal antibodies.

WO2004/032841 discloses a system for production and screening of monoclonal antibodies.

Tiller *et al* disclose efficient generation of monoclonal antibodies from single human B cells by single cell RT-PCT and expression vector cloning.

Babcook et al 1996, Proc. Natl. Acad. Sci, 93, 7843-7848 discloses a novel strategy for generating monoclonal antibodies from single, isolated lymphocytes producing antibodies of defined specificities.

Coloma et al Journal of Immunological Methdos vol 152, no. 1, 31 July 1992, pages 89-104.

WO 92/02551 discloses a method for producing proteins with desired function which enables a single lymphocyte that is producing an antibody with a desired specificity to be identified within a large population of lymphoid cells and the genetic information that encodes the specificity of the antibody (the variable regions) to be identified from that lymphocyte. Following identification of the amino acid sequence of the variable regions or portions thereof the sequences may be used to construct another antibody with the desired function by cloning the DNA sequences encoding the variable regions into a vector capable of expressing the protein. The speed and output capacity of this method is limited by a number of factors particularly by the variable region cloning into vectors.Accordingly, there is a need to provide improved methods for producing proteins having a desired function, particularly antibodies, which overcome the limitations of methods currently used in the art.

### Summary of Invention

It is an aim of the present invention to solve one or more of the problems described above.

The present disclosure provides a method for obtaining a recombinant antibody with a desired ability to bind a selected antigen which avoids multiple digestion and ligation steps to produce vectors and multiple transformations into host cells, comprising:
(a) providing a population of antibody-forming lymphocyte cells suspected of containing at least one cell capable of producing an antibody comprising a heavy chain and a light chain exhibiting said desired specificity;
   (aa) a first screening step prior to step (b) in which the population of antibody-forming cells provided in step (a) are screened to identify an antibody-forming cell or a population of antibody-forming cells producing an antibody exhibiting the desired ability;
   (aaa) identifying and isolating a single antibody-forming cell producing an antibody exhibiting the desired ability from a population of antibody-forming cells identified by the first screening step (aa);
   (b) generating a transcriptionally active recombinant linear polynucleotide encoding a light chain or a fragment thereof and a transcriptionally active recombinant linear polynucleotide encoding a heavy chain or a fragment thereof for each recombinant antibody from the single isolated antibody forming cell obtained in step (aaa), wherein each transcriptionally active recombinant linear polynucleotide comprises a first encoding polynucleotide sequence encoding a variable domain of an antibody produced by the single isolated antibody-forming cell obtained in step (aaa), a second encoding polynucleotide sequence encoding one or more constant domain encoding polynucleotide sequences and one or more transcription regulatory elements and is not capable of autonomous replication; wherein step (b) comprises:
      bb) providing the first encoding polynucleotide sequence, the second encoding polynucleotide sequence and the one or more transcription regulatory elements;
      bbb) fusing the first encoding polynucleotide sequence and the second encoding polynucleotide sequence by PCR, and
      bbbb) fusing one or more transcription regulatory elements to the first encoding polynucleotide sequence and/or the second encoding polynucleotide sequence by PCR;
   (c) expressing a recombinant antibody comprising a heavy chain and a light chain from the transcriptionally active recombinant linear polynucleotides generated in step (b) by transiently transfecting the transcriptionally active recombinant linear polynucleotides into a cell, growing said host cells in an appropriate medium and isolating the antibody from the cell;
   (d) screening the recombinant antibody produced by step (c) for the desired specificity to bind a selected antigen; and
   (e) optionally repeating steps (b), (c) and (d) to identify a recombinant antibody exhibiting the desired specificity to bind a selected antigen.

The methods provided by the present invention are advantageous because they provide a more efficient, simplified and quicker way to produce a protein, particularly fusion proteins which assemble to form a multimeric protein, such as an antibody. The transcriptionally active recombinant linear polynucleotide, cell, expression system, and the methods avoid the need for multiple digestion and ligation steps to produce vectors, multiple transformations into host cells, and allow a faster and easier way to obtain the final recombinant protein.

The cell employed according to the present invention may also be capable of being transfected with a greater number of exogenous linear transcriptionally active polynucleotides compared to the number of plasmids. Accordingly, the method of the present invention may provide a more efficient transfection and expression method and may provide a higher yield of expressed protein compared to the use of plasmids.

### Brief Description of the Drawings

In the following drawings specific embodiments of the present invention are described by way of example only, in which:

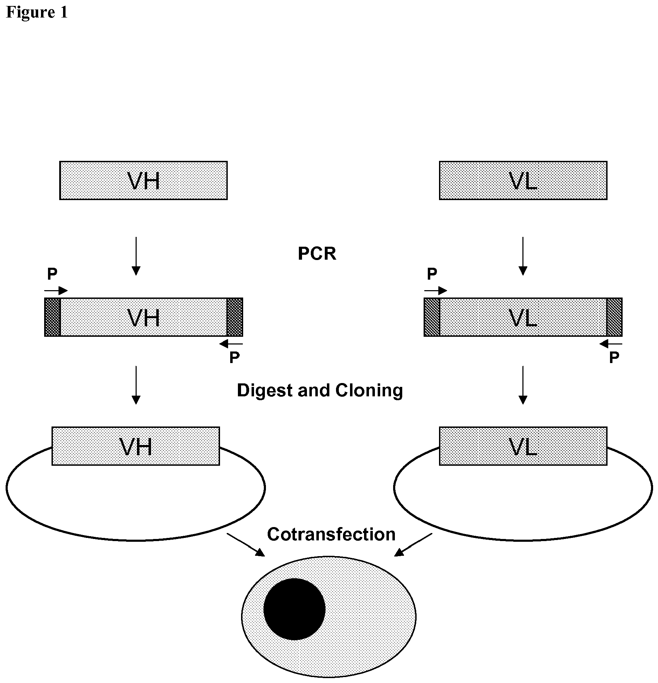

Figure 1 is a schematic diagram of a known standard method used to clone and express antibody variable regions using expression vectors wherein the variable heavy chain (VH) and variable light chain (VL) encoding sequences are amplified by PCR in the presence of primers (P) which add restrictions sites at the 5' and 3' ends of the VH and VL sequences which are shown as shaded regions either side of the VH and VL sequences; the restriction sites allow downstream digestion and ligation into separate expression vectors which may comprise downstream constant domain sequences; the two expression vectors are then transfected into a cell to provide a transient or stably transfected cell capable of expressing the desired antibody.

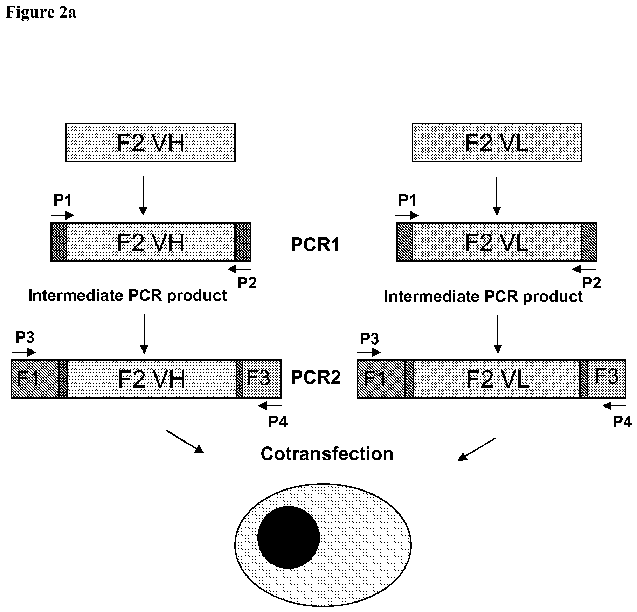

Figure 2a is a schematic diagram of the method for producing two distinct exogenous linear transcriptionally active polynucleotides wherein the variable heavy chain (F2 VH) and variable light chain (F2 VL) encoding sequences are amplified in a first PCR (PCR1) in the presence of overlap extension primers P1 and P2 which add the 3' end of the first transcription regulatory element (F1) to the 5' end of the encoding polynucleotide sequence (F2) and the 5' end of the 3' end polynucleotide sequence (F3) to the 3' end of the encoding polynucleotide sequence (F2) thereby producing an intermediate PCR product; in a second PCR (PCR2) the intermediate PCR product is amplified by PCR in the presence of a first transcription regulatory element (F1), a 3' end polynucleotide sequence (F3), a third primer (P3) and a fourth primer (P4) thereby producing a transcriptionally active recombinant linear polynucleotide comprising the first transcription regulatory element (F1) and the encoding polynucleotide sequence (F2) and the 3'end polynucleotide sequence (F3); the VH transcriptionally active recombinant polynucleotide sequence and VL transcriptionally active recombinant polynucleotide may then be transfected into a cell to provide a transient or stably transfected cell capable of expressing the desired antibody or fragment thereof.

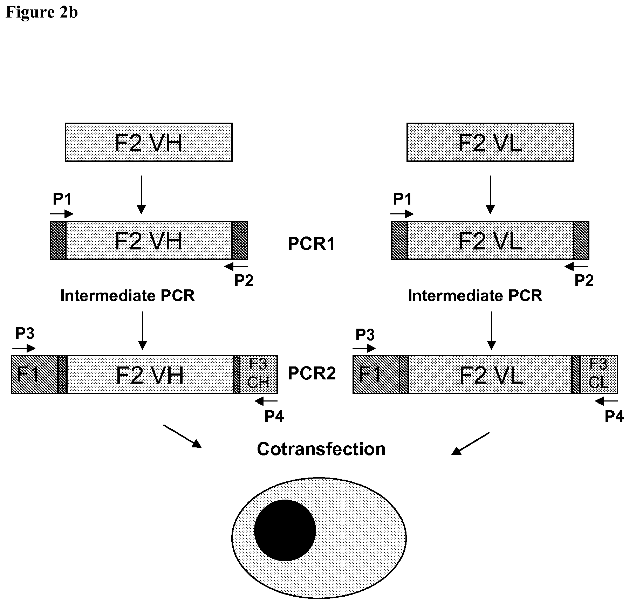

Figure 2b is a schematic diagram of the method for producing recombinant linear transcriptionally active polynucleotides encoding a heavy chain or light chain of an antibody, wherein the variable heavy chain (F2 VH) encoding sequence is amplified in a first PCR (PCR1) in the presence of overlap extension primers P1 and P2 which add the 3' end of the first transcription regulatory element (F1) to the 5' end of the variable heavy chain encoding polynucleotide sequence (F2) and the 5' end of the constant heavy chain sequence (F3 CH) to the 3' end of the variable heavy chain encoding polynucleotide sequence (F2 VH) thereby producing an intermediate PCR product; in a second PCR (PCR2) the intermediate PCR product is amplified by PCR in the presence of a first transcription regulatory element (F1), a constant heavy chain sequence (F3 CH), a third primer (P3) and a fourth primer (P4) thereby producing a transcriptionally active recombinant linear polynucleotide comprising the first transcription regulatory element (F1) and the variable heavy chain encoding polynucleotide sequence (F2 VH) and the constant heavy chain encoding polynucleotide sequence (F3 CH); PCR1 and PCR2 are repeated with a variable light chain encoding sequence (F2 VL) and a constant light chain encoding sequence (F3 CL) to produce a transcriptionally active recombinant linear polynucleotide comprising the first transcription regulatory element (F1) and the variable light chain encoding polynucleotide sequence (F2 VL) and the constant light chain encoding polynucleotide sequence (F3 CL); the heavy chain transcriptionally active recombinant polynucleotide sequence and light chain transcriptionally active recombinant polynucleotide may then be transfected into a cell to provide a transient or stably transfected cell capable of expressing the desired antibody or fragment thereof.

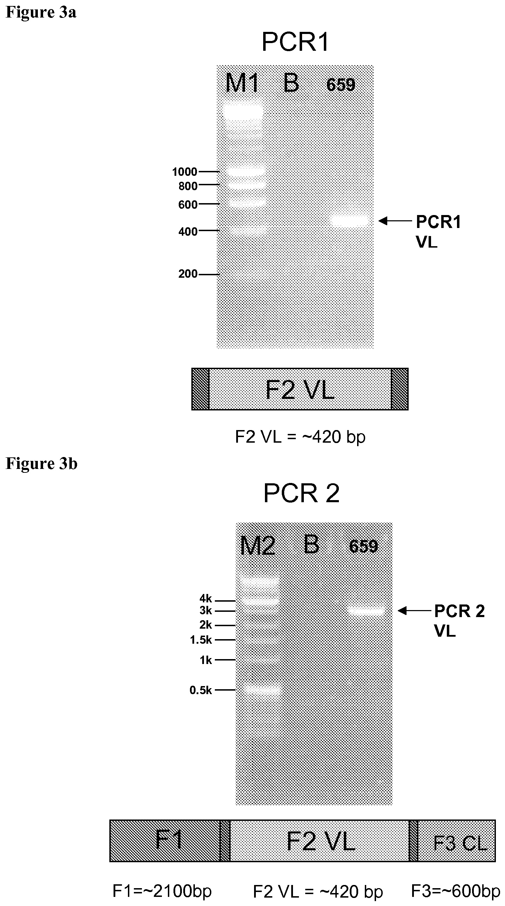

Figure 3a shows an SDS Page of the intermediate PCR product (PCR1 VL product) of the first PCR1 for the variable light chain (F2 VL), wherein M1 is a standard DNA ladder, B is a blank negative control and 659 is the VL intermediate PCR product.
Figure 3b shows an SDS Page of the final PCR2 product (PCR2 VL product) of the second PCR2 for the variable light chain (VL) comprising a promoter sequence (F1), the variable light chain sequence (F2 VL) and the constant light chain sequence and poly A sequence (F3 CL), wherein M2 is a standard DNA ladder, B is a blank negative control and 659 is the final PCR product.

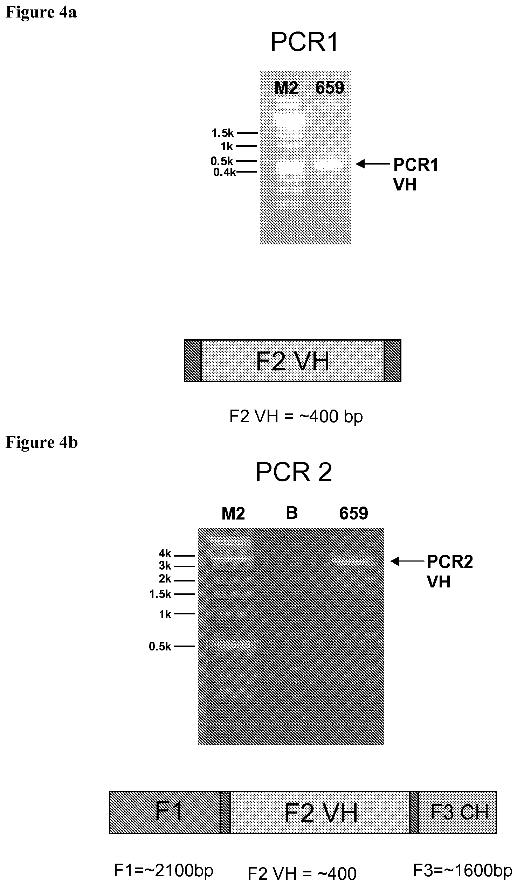

Figure 4a shows an SDS Page of the intermediate PCR product (PCR1 VH product) of the first PCR1 for the variable heavy chain (F2 VH), wherein M2 is a standard DNA ladder, B is a blank negative control and 659 is the VH intermediate PCR product.
Figure 4b shows an SDS Page of the final PCR2 product (PCR2 VH product) of the second PCR2 for the variable heavy chain (VH) comprising a promoter sequence (F1), the variable heavy chain sequence (F2 VH) and the constant heavy chain sequence and poly A sequence (F3 CH), wherein M2 is a standard DNA ladder, B is a blank negative control and 659 is the final PCR product.

### Brief Summary of the Sequences

SEQ ID NO: 1 is the nucleotide sequence of a second primer (P2) comprising a region complementary to the 3' end of a variable heavy chain domain sequence (F2) and an overlap-extension tail complementary to the 5' end of a constant heavy chain domain sequence (F3).

SEQ ID NO: 2 is the amino acid sequence encoded by SEQ ID NO: 1 of the second primer (P2), wherein QGTLVTVSS is the 3' end of the variable heavy chain domain (F2) and ASTKGP is the 5' end of the constant heavy chain domain sequence (F3).

SEQ ID NO: 3 is the nucleotide sequence of a first primer (P1) comprising a region complementary to the 5' end of a variable heavy chain domain containing sequence (F2) and an overlap-extension tail complementary to the 3' non-coding end of a promoter sequence (F1).

SEQ ID NO: 4 is the amino acid sequence of the 5' end of the variable heavy chain domain containing sequence (F2) encoded by SEQ ID NO: 3 of the first primer (P1).

SEQ ID NO: 5 is the nucleotide sequence of a second primer (P2) comprising a region complementary to the 3' end of a variable light chain domain sequence and an overlap-extension tail complementary to the 5' end of a constant light chain domain sequence (F3).

SEQ ID NO: 6 is the amino acid sequence encoded by SEQ ID NO: 5 of the second primer, wherein GTKVEIKR is the 3' end of the variable light chain domain and TVAAPSVF is the 5' end of the constant light chain domain sequence (F3).

SEQ ID NO: 7 is the nucleotide sequence of a first primer (P1) comprising a region complementary to the 5' end of a variable light chain domain containing sequence (F2) and an overlap-extension tail complementary to the 3' non-coding end of a promoter sequence (F1).

SEQ ID NO: 8 is the amino acid sequence of the 5' end of the variable light chain domain containing sequence (F2) encoded by SEQ ID NO: 7 of the first primer (P1).

SEQ ID NO: 9 is the nucleotide sequence of a fourth primer (P4) which is complementary to a non-coding region at the 3' end of the polyadenylation sequence of F3.

SEQ ID NO: 10 is the nucleotide sequence of a third primer (P3) which is complementary to a non-coding region at the 5' end of the promoter sequence (F1).

SEQ ID NO: 11 is the nucleotide sequence comprising the sequence of hCMV promoter (F1).

SEQ ID NO: 12 is the nucleotide sequence comprising a constant mouse Kappa chain sequence and a polyA sequence.

SEQ ID NO: 13 is the nucleotide sequence comprising a constant mouse gamma 1 chain sequence and a polyA sequence.

### Detailed Description of the Invention

The present invention will now be described in more detail.

The terms "protein" and "polypeptide" are used interchangeably herein, unless the context indicates otherwise. "Peptide" is intended to refer to 10 or less amino acids.

The terms "polynucleotide" includes a gene, DNA, cDNA, RNA, mRNA etc unless the context indicates otherwise. The polynucleotide may be double or single stranded.

As used herein, the term "comprising" in context of the present specification should be interpreted as "including".

The term "distinct" in the context of the present invention means that the polynucleotides are different/non-identical. The polynucleotides may, for example, have 95% or less homology, 90% or less homology, 85% or less homology, 80% or less homology or 75% or less homology when the full-length sequences are compared, for example, using suitable software such as BLAST.

The term "exogenous" in the context of the present invention means that polynucleotide sequences originated from outside the host cell.

The term "fusion protein" in the context of the present invention means a polypeptide comprising a plurality of subunits wherein each subunit is encoded by a separate encoding polynucleotide sequence. Each encoding polynucleotide sequence comprises one or more gene sequences or fragments thereof. Translation of the plurality of encoding polynucleotide sequences results in a single polypeptide chain. The subunits of the fusion protein may be linked directly in the polypeptide chain or linked via a suitable linker sequence. Typically, the fusion protein is a heavy chain or light chain of an antibody comprising a variable domain and a constant domain or fragments thereof.

The term "transcriptionally active recombinant linear polynucleotide" in the context of the present invention is a linear polynucleotide comprising the necessary elements required for transcription of the coding sequences in the polynucleotide after transfection into the host cell. The linear transcriptionally active polynucleotide comprises one or more regulatory expression sequences. The one or more regulatory expression sequences may include a promoter. The one or more regulatory expression sequences may include a 3' untranslated region such as a polyadenylation sequence and/or a termination sequence. In one embodiment, each linear transcriptionally active polynucleotide comprises a promoter, a plurality of protein coding sequences and a polyadenylation sequence.

Traditionally, polynucleotides are handled in the form of plasmids. This may be because the plasmid provides a relatively stable form in which the relevant polynucleotide can be handled, stored, manipulated and the like. Plasmid DNA is not linear transcriptionally active polynucleotide within the context of the present specification, but may be cleaved to provide linear transcriptionally active polynucleotide. In one embodiment the transcriptionally active polynucleotide does not comprise backbone vector sequences. In one embodiment the transcriptionally active polynucleotide is not capable of autonomous replication, for example the transcriptionally active polynucleotide does not comprise an origin of replication.

Each linear transcriptionally active polynucleotide may be in the form of a transcriptionally active DNA fragment as described in US 6,280,977 and Liang, X et al, 2002, The Journal of Biological Chemistry, 227(5), 3593-3598, which disclose transcriptionally active PCR (TAP) fragments used for expression experiments.

Whilst US 6,280,977 and Liang, X et al, 2002, The Journal of Biological Chemistry, 227(5), 3593-3598 disclose a method for producing a linear transcriptionally active polynucleotide and the use of a linear transcriptionally active polynucleotide for expressing a protein, it is not described or suggested that each linear transcriptionally active polynucleotides may comprise a plurality of encoding polynucleotide sequences wherein each encoding polynucleotide sequence encodes a subunit of a fusion protein, such as a heavy chain or light chain of an antibody or a fragment thereof. Further it is not described or suggested that two distinct linear transcriptionally active polynucleotides may be transfected in a cell to express distinct polypeptides, particularly distinct polyptpides which assemble to form multimeric proteins, such as antibodies.

The linear transcriptionally active polynucleotide comprises one or more transcription regulatory elements and an encoding polynucleotide sequence. In one embodiment the linear transcriptionally active polynucleotide comprises a plurality of encoding polynucleotide sequences.

The linear transcriptionally active polynucleotide may be any suitable nucleic acid sequence such as DNA or RNA.

The component sequences of the polynucleotide including the one or more regulatory expression sequences and one or more protein coding sequences may be separated by non-coding/non-functional nucleotide sequences. In the embodiment wherein a plurality of protein encoding sequences are present, wherein each encoding sequence encodes a subunit of a fusion protein, preferably a single polypeptide chain is formed after translation. Each protein encoding sequence may be separated by one or more non-coding sequences such as introns, which may be removed by splicing of the mRNA. In one embodiment the coding polynucleotide sequences are separated by a linker sequence, which preferably encodes a polypeptide linker. The polypeptide linker may be any suitable length in order to allow the subunits of the fusion protein to assemble into a functional fusion protein. In one embodiment the coding polynucleotide sequences are not separated by non-coding sequences or linker sequences and are contiguously linked.

In one embodiment the linear transcriptionally active polynucleotide comprises a first encoding polynucleotide sequence (F2), a first transcription regulatory element (F1) at the 5' end of the first encoding polynucleotide sequence (F2) and a 3' end sequence (F3) at the 3' end of the encoding polynucleotide sequence (F2). The 3' end polynucleotide sequence (F3) may comprise a second transcription regulatory element and/or a second encoding polynucleotide sequence.

Any suitable one or more encoding polynucleotide sequences may be employed in the one or more linear transcriptionally active polynucleotides. The encoding sequence may be the minimum sequence encoding a protein of interest, such as a subunit of a fusion protein i.e. which consists essentially of the nucleotide sequences encoding the relevant polypeptide. Each encoding polynucleotide sequence encodes one or more genes or fragments thereof.

Each encoding polynucleotide sequence may be identical to an endogenous polynucleotide sequence encoding a protein or a mutated version thereof, for example with attenuated biological activity, or fragment thereof. Alternatively, each encoding sequence employed in the present invention encodes a heterologous protein, not normally expressed by the host cell.

In one embodiment each encoding polynucleotide sequence is autologous. Alternatively, each encoding polynucleotide sequence may be heterologous.

The linear transcriptionally active polynucleotide may comprise any suitable number of encoding polynucleotide sequences, which preferably form a fusion protein. Preferably the linear transcriptionally active polynucleotide comprises 2 encoding polynucleotide sequences, 3 encoding polynucleotide sequences or 4 encoding polynucleotide sequences.

In one embodiment the cell according to the present invention comprises two linear transcriptionally active polynucleotides wherein each polynucleotide comprises a plurality of encoding polynucleotide sequences each encoding a subunit of a fusion protein. Each linear transcriptionally active polynucleotide may comprise a plurality of identical encoding polynucleotide sequences and/or a plurality of distinct encoding polynucleotide sequences. The encoding polynucleotide sequences may encode a fusion protein of interest which may be any suitable fusion protein including therapeutic, prophylactic or diagnostic proteins.

The one or more proteins expressed may also contain a tag or label to assist in purification/ separation.

The linear transcriptionally active polynucleotide may comprise one or more further encoding polynucleotides sequences for expression which form one or more separate polypeptide chains after translation. In one embodiment the linear transcriptionally active polynucleotide comprises a plurality of further encoding polynucleotides, wherein each encoding polynucleotide sequence encodes a subunit of one or more further fusion proteins. For example, a linear transcriptionally active polynucleotide may comprise a plurality of encoding polynucleotide sequences encoding a light chain of an antibody and a plurality of encoding polynucleotide sequences encoding heavy chain of an antibody.

The cell according to the present invention may also comprise one or more further distinct linear transcriptionally active polynucleotides, wherein each further linear transcriptionally active polynucleotide comprises a polynucleotide sequence encoding a distinct protein, preferably a plurality of encoding polynucleotide sequences each encoding a subunit of a fusion protein as defined above. Preferably the cell comprises a plurality of linear transcriptionally active polynucleotides, wherein each polynucleotide comprises distinct plurality of encoding polynucleotide sequences encoding distinct fusion proteins for expression.

In the embodiment wherein a plurality of distinct proteins are encoded by one linear transcriptionally active polynucleotide and/or by a plurality of distinct linear transcriptionally active polynucleotides, the distinct proteins may be unrelated proteins. These proteins may not be co-assembled and may be separated by routine techniques, after expression.

In a preferred embodiment, the protein, preferably the fusion protein, is a component of a multimeric protein. In the embodiment wherein a plurality of distinct fusion proteins are encoded by one linear transcriptionally active polynucleotide and/or by a plurality of distinct linear transcriptionally active polynucleotides, preferably each distinct protein is a component of a multimeric protein wherein each component of the multimeric protein may associate to form the multimeric protein, for example by covalent and/or non-covalent bonds. Examples of multimeric proteins include proteins that comprise an alpha and beta chain such as hemoglobin, integrin and the like.

The multimeric protein may be formed from a plurality of identical and/or distinct fusion proteins expressed by transcriptionally active linear polynucleotides according to the present invention.

In this embodiment the transcriptionally active linear polynucleotides may encode protein components of a multimeric protein which form a cognate pair derived from a single cell. Accordingly, expression of the proteins from the transcriptionally active polynucleotides in the cell ensures that the original activity of the multimeric protein is maintained.

In this embodiment the cell expresses the multimeric protein preferably in an assembled form. Accordingly, a further advantage of the present invention is that the multimeric protein produced is assembled in the cell and may have the desired conformation required for *in vivo* activity.

In the present invention the one or more proteins expressed by one or more of the transcriptionally active linear polynucleotide sequences is an antibody or a fragment thereof.

Antibodies in the context of the present invention include whole antibodies of any suitable class for example, IgA, IgD, IgE, IgG or IgM or subclass such as IgG1, IgG2, IgG3 or IgG4 and functionally active fragments or derivatives thereof and may be, but are not limited to, monoclonal, humanised, fully human or chimeric antibodies.

Antibodies may therefore comprise a complete antibody molecule having full length heavy and light chains or a fragment thereof and may be, but are not limited to VH, VL, VHH, Fab, modified Fab, Fab', F(ab')₂, Fv, scFv, bi, tri or tetra-valent antibodies, Bis-scFv, diabodies, triabodies, tetrabodies and epitope-binding fragments of any of the above (see for example Holliger and Hudson, 2005, Nature Biotech. 23(9):1126-1136; Adair and Lawson, 2005, Drug Design Reviews - Online 2(3), 209-217). Other antibody fragments include the Fab and Fab' fragments described in International patent applications WO2005/003169, WO2005/003170 and WO2005/003171. Multi-valent antibodies may comprise multiple specificities or may be monospecific (see for example WO 92/22853 and WO05/113605).

The constant domains of the antibody molecule, if present, may be selected having regard to the proposed function of the antibody molecule, and in particular the effector functions which may be required. For example, the constant region domains may be human IgA, IgD, IgE, IgG or IgM domains. In particular, human IgG constant region domains may be used, especially of the IgG1 and IgG3 isotypes when the antibody molecule is intended for therapeutic uses and antibody effector functions are required. Alternatively, IgG2 and IgG4 isotypes may be used when the antibody molecule is intended for therapeutic purposes and antibody effector functions are not required, e.g. for simply blocking activity. It will be appreciated that sequence variants of these constant region domains may also be used.

Accordingly, in one embodiment of the present invention each encoding polynucleotide sequence encodes one or more antibody domains or fragments thereof. "Antibody domain" in the context of the present invention means a variable or constant domain or fragment thereof of an antibody. The term "region" may be used interchangeably with the term "domain". Examples of antibody domains include VH, VL, VHH, IgNAR variable domains, CL, CH1, CH2 and CH3 constant domains. Reference to "the constant heavy chain domain" or "one or more constant heavy chain domains" may refer to one or more domains of CH1, CH2 and CH3. Any selected encoding polynucleotide sequence may comprise a whole antibody domain, comprise a fragment of an antibody domain or comprise two or more antibody domains or fragments thereof. Accordingly, each encoding polynucleotide sequence may encode a variable domain, or fragment thereof, and/or one or more constant domains, or fragment thereof, of a heavy or light chain from an antibody. Therefore, each transcriptionally active polynucleotide may encode a variable domain or one or more constant domains or a fusion protein of a variable domain and constant domain.

In one embodiment, one or more of the linear transcriptionally active polynucleotides may each comprise two or more encoding polynucleotide sequences encoding separate polypeptide sequences. For example, a linear transcriptionally active polynucleotide may comprise a heavy chain variable domain sequence and a light chain variable domain sequence or a full light chain sequence and a full heavy chain sequence.

The one or more expressed proteins may be single chains which do not assemble to form a multimeric antibody protein. Alternatively, in the embodiment wherein a plurality of proteins are expressed the proteins may assemble into a multimeric antibody protein comprising a plurality of identical and/or distinct proteins.

Any combination of one or more domains or fragments thereof may be encoded by each encoding polynucleotide sequence.

In one embodiment the protein expressed is a scFv formed from a plurality of variable domain sequences. The transcriptionally active linear polynucleotide sequence may comprise two encoding polynucleotide sequences wherein each encoding polynucleotide sequence comprises a variable domain selected from VH, VL, VHH, IgNAR variable domains. The selected variable domains in each encoding polynucleotide sequence may be the same or different domains. For example, the expression fusion protein may be VH-VH, VL-VL, VH-VL or VH-VL. The subunits of the fusion protein may be linked by a polypeptide linker sequence.

One or more linear transcriptionally active polynucleotides encode a light chain or fragment thereof.

One or more linear transcriptionally active polynucleotides encodes a heavy chain from an antibody or a fragment thereof.

In the present method, one or more linear transcriptionally active polynucleotides encodes a light chain from an antibody or a fragment thereof and one linear transcriptionally active polynucleotide encodes a heavy chain from an antibody or a fragment thereof. Accordingly, two linear transcriptionally active polynucleotides may together encode an antibody or a fragment thereof. In an alternative embodiment a linear transcriptionally active polynucleotide encodes both the light chain or fragment thereof and the heavy chain or fragment thereof.

Preferably the light chain or fragment thereof and/or heavy chain or fragment thereof comprises a variable domain or fragment thereof and one or more constant domains or fragments thereof. For example, a linear transcriptionally active polynucleotide may comprise a plurality of encoding polynucleotide sequences wherein:
- a first encoding polynucleotide sequence encodes a VH domain and a second encoding polynucleotide sequence encodes a CH1 domain; or
- a first encoding polynucleotide sequence encodes a VH domain and a CH1 domain and a second encoding polynucleotide sequence encodes a CH2 domain and CH3 domain; or
- a first encoding polynucleotide sequence encodes a VH domain and a second encoding polynucleotide sequence encodes a CH1 domain; or
- a first encoding polynucleotide sequence encodes a VH domain, a second encoding polynucleotide sequence encodes a CH1 domain, a third encoding polynucleotide sequence encodes a CH2 domain and a fourth encoding polynucleotide sequence encodes a CH3 domain.

In a preferred embodiment, wherein a plurality of distinct fusion proteins are encoded by one linear transcriptionally active polynucleotide and/or by a plurality of distinct linear transcriptionally active polynucleotides, the polynucleotide(s) may encode a light chain from an antibody or a fragment thereof and a heavy chain from an antibody or a fragment thereof, as defined above. The light chain and heavy chain polypeptide sequences encoded by the transcriptionally active polynucleotide(s) may assemble to form a multimeric antibody or a fragment thereof after expression e.g. a Fab or Fab' fragment.

Each antibody domain may be derived from the same or different source. Accordingly, in the embodiment wherein the linear transcriptionally active polynucleotide encodes a variable domain and one or more constant domains from a light chain or heavy chain, the variable domains and constant domains may be heterologous. For example, the variable domains, or fragments thereof, may be derived from a mouse and the constant domains may be human.

In the embodiment wherein the linear transcriptionally active polynucleotide comprises a first encoding polynucleotide sequence (F2), a first transcription regulatory element (F1) at the 5' end of the encoding polynucleotide sequence (F2) and a 3' end sequence (F3) at the 3' end of the encoding polynucleotide sequence (F2). The 3' end polynucleotide sequence (F3) may comprise a second transcription regulatory element and/or a second encoding polynucleotide sequence. In the embodiment wherein F3 comprises a second encoding polynucleotide sequence, the first encoding polynucleotide sequence (F2) may encode a variable domain or fragment thereof of an antibody and the second encoding polynucleotide sequence (F3) may comprise a sequence encoding one or more constant domains or fragment thereof of an antibody. The second encoding polynucleotide sequence (F3) may also comprise a second transcription regulatory element, such as a polyadenylation sequence.

In the embodiments wherein each fusion protein is formed from a light chain variable domain sequence and a heavy chain variable domain sequence or a plurality of distinct fusion proteins each comprising a heavy chain or light chain variable domain sequence are encoded by one linear transcriptionally active polynucleotide and/or by a plurality of distinct linear transcriptionally active polynucleotides, the variable heavy chain sequence and variable light chain sequence may form a cognate pair from a single cell, thereby maintaining the binding affinity of the endogenous antibody from the cell. In a further embodiment, the variable heavy chain sequence and the variable light chain sequence do not form a cognate pair. In this embodiment the variable heavy chain sequences and variable light chain sequences may be randomly paired sequences derived from a population of genetically diverse cells.

After expression of the antibody or fragment thereof may be further processed, for example by conjugation to another entity or for example PEGylated to generate a product with the required properties.

In one aspect the antibody or fragment thereof produced is useful in therapy or diagnosis, research, analysis, assays or the like. The antibodies of the present disclosure may possess various modifications. For example, the antibodies may be conjugated to one or more reporter or effector molecules, tags or labels such a radiolabel, luminescent/fluorescent label, for any suitable diagnostic or therapeutic purpose.

The linear transcriptionally active polynucleotide of the present invention comprises one or more transcription regulatory elements.

Preferably the linear transcriptionally active polynucleotide comprises a first transcription regulatory element comprising a promoter sequence. As used herein, the term "promoter" is a DNA sequence which generally extends upstream from the transcription initiation site and is involved in binding of RNA polymerase. The promoter may contain several short (<10 base pair) sequence elements that bind transcription factors, generally dispersed over >200 base pairs. A promoter that contains only elements recognized by general and upstream factors is usually transcribed in any cell type. Such promoters may be responsible for expression of cellular genes that are constitutively expressed (sometimes called housekeeping genes). There are also tissue-specific promoters limited to particular cell types, such as the human metallothionein (MT) promoter which is upregulated by heavy metal ions and glucocorticoids. Any suitable promoter sequence may be employed in the present invention depending on the type of host cell employed. Examples of suitable promoters include CMV such as hCMV, lac (a bacterial cell promoter), viral LTR promoters, SV40 promoter, elongation factor promoter (EFA1) and immediate early promoter IE1 for insect host cells. Any other baculovirus promoters that are expressed independent of the baculovirus gene expression could also be used such as for example the gp64 promoter. One or more promoters employed may be inducible promoters.

In the embodiment wherein the cell comprises a plurality of linear transcriptionally active polynucleotides, each polynucleotide may comprise the same or different promoters. If each linear transcriptionally active polynucleotide comprises different promoters, the strength of the promoters may be advantageously selected in order to selectively up-regulate or down-regulate the expression of one linear transcriptionally active polynucleotide compared to another linear transcriptionally active polynucleotide.

The linear transcriptionally active polynucleotide may comprise an enhancer sequence upstream of the promoter sequence. For example, a homologous region 5 (hr5) enhancer sequence may be positioned upstream of an immediate early promoter (IE1) promoter sequence for insect host cells.

The linear transcriptionally active polynucleotide may be transcribed by cells without the presence of a terminator sequence. Thus, in one aspect each linear transcriptionally active polynucleotide does not comprise a terminator.

In a further embodiment each linear transcriptionally active polynucleotide comprises a second transcription regulatory element.

The second transcription regulatory element is preferably any suitable DNA sequence that is capable of forming the end of the RNA, including mRNA or pre-mRNA, transcript formed from the encoding polynucleotide sequence. The regulatory sequence may form the end of the RNA transcript by terminating transcription and/or initiating cleavage of the RNA transcript.

In the embodiment wherein the regulatory sequence is a transcription terminator, the sequence typically causes the RNA polymerase to cease transcription by forming a stem-loop structure in the RNA. The stem-loop structure causes the RNA polymerase to fall away from the DNA, therefore, terminating transcription. Any suitable transcription terminator may be used depending on the type of host cell employed. An immediate early IE1 terminator sequence may be used for insect host cells.

In the embodiment wherein the regulatory sequence initiates cleavage of the RNA transcript, the sequence in the RNA transcript causes a protein to bind the RNA transcript and cleave it, thereby forming the end of the RNA transcript. A suitable sequence is a polyadenylation sequence which is transcribed in the RNA where it causes an enzyme to bind and catalyze cleavage of the RNA and also causes an enzyme to add A residues at the 3' end, thereby forming a polyadenylation tail. The polyadenylation sequence initiates cleavage of the RNA transcript and may protect the mRNA from exonucleases thereby stabilizing mRNA production. Examples of polyadenylation sequences include SV40poly A, BgH polyA and synthetic polyA sequence.

In one embodiment the regulatory sequence terminates transcription and initiates cleavage of the RNA transcript. For example, it is thought that the SV40polyA sequence is capable of initiating cleavage of the RNA transcript and also terminating transcription by RNA polymerase.

In one embodiment the transcriptionally active polynucleotide sequence comprises a transcription terminator and a polyadenylation sequence. In an alternative embodiment the transcriptionally active polynucleotide sequence comprises a polyadenylation sequence but does not comprise a separate transcription terminator sequence.

In the embodiment wherein the cell comprises a plurality of linear transcriptionally active polynucleotides, each linear transcriptionally active polynucleotide may comprise the same or different sequence that is capable of forming the end of the RNA transcript.

The linear transcriptionally active polynucleotide may comprise one or more introns. In one embodiment one or more of the polynucleotide sequences comprise one or more introns within each encoding polynucleotide sequence and/or between each encoding polynucleotide sequence.

In one embodiment each encoding polynucleotide sequence comprises 1, 2 or 3 introns.

The intron may be derived from any gene, particularly a gene from which the encoded sequence is derived.

In one embodiment the linear transcriptionally active polynucleotide comprises a Kozak sequence, for example the natural sequence associated with sequence encoding the desired polypeptide. Whilst not wishing to be bound by theory, it is thought that a Kozak sequence in the mRNA, may have a role to play in assembling the ribosome for translation of the mRNA.

In one embodiment the linear transcriptionally active polynucleotide sequence may comprise an internal ribosome entry site (IRES) sequence which allows translation initiation in the middle of mRNA. The use of one or more IRES sequences in the linear transcriptionally active polynucleotide sequences may be particularly useful in the embodiment wherein the linear transcriptionally active polynucleotides comprises one or more further encoding polynucleotide sequences, preferably a plurality of further polynucleotides sequences each encoding a subunit of a fusion protein. An IRES sequence may be positioned between encoding polynucleotide sequences, such as between a heavy chain encoding sequence and a light chain encoding sequence, to allow separate translation of the mRNA to produce the encoded polypeptide sequences.

The ends of linear transcriptionally active polynucleotide may comprise peptide nucleic acid (PNA) tails, claims and/or "clamp tails" to protect the ends of the polynucleotide from digestion by exonucleases after the polynucleotides have been transfected into a cell. Suitable PNAs are described in US 6,280,977. Alternatively, in one embodiment the linear transcriptionally active polynucleotide does not comprise or is not attached to a PNA including a PNA tail, PNA clamp, PNA "claim tail" or PNA molecule.

The present invention also provides a cell comprising one or more transcriptionally active recombinant linear polynucleotides as defined above. As discussed previously, the cell may comprise a plurality of transcriptionally active recombinant linear polynucleotides as defined above wherein each polynucleotide encodes a distinct fusion protein, such as a heavy chain or fragment thereof of an antibody and a light chain or fragment thereof of an antibody.

Any cell able to express the protein encoded by the linear transcriptionally active polynucleotide may be employed in the present invention. Suitable cells for employing in the invention include eukaryotic cells, for example plant cells, insect cells such as *Spodoptera frugiperda* Sf9, *Spodoptera frugiperda* Sf21 and *Trichoplusia ni* Tni, yeast cells, animal cells such as mammalian cells, in particular CHO cells, myeloma cells, viro cells, MRC5 Cells, HEK cells, African green monkey COS cells, human PerC6 cells and the like.

In some instances, it is desirable to employ a mammalian cell because it may produce the protein product with appropriate conformation required for biological activity. Tertiary structure and conformation can be vitally important because incorrect tertiary structure or conformation can result in loss of some or all biological activity. CHO cells have been found to be particularly useful for mammalian expression because the proteins expressed in CHO cells have glycoforms that are generally compatible and bioactive in humans. Accordingly, in one aspect the invention employs a mammalian cell such as a CHO cell, for example a CHOS cell (Invitrogen Cat. No. 11619-012, Deaven, L.L. et al, 1973, Chromasoma 41, 129, D'Anna, J.A. et al, 1996, Methods in Cell Science 18, 115, D'Anna, J.A. et al, 1997, Radiation Research 148, 260) or a derivative therefrom.

The present invention also provides a method of expressing a protein, comprising:
a) transfecting a cell with one or more transcriptionally active recombinant linear polynucleotides, as described above, and
b) expressing the one or more proteins, preferably fusion proteins, encoded by the one or more transcriptionally active recombinant linear polynucleotides.

In a preferred embodiment, step a) comprises transfecting the cell with a first transcriptionally active recombinant linear polynucleotide encoding a heavy chain or fragment thereof of an antibody and a second transcriptionally active recombinant linear polynucleotide encoding a light chain or fragment thereof of an antibody and/or a transcriptionally active recombinant linear polynucleotide encoding both a heavy chain or fragment thereof of an antibody and a light chain or fragment thereof of an antibody; and step b) comprises expressing the heavy chain polypeptide or fragment thereof and light chain polypeptide or fragment thereof. In this embodiment, the heavy chain polypeptide and the light chain polypeptide preferably assemble to form an antibody or fragment thereof.

The method may also comprise a step of isolating the antibody or fragment thereof from the cell.

The linear transcriptionally active polynucleotide can be incorporated into a cell in various ways using routine means known for transfecting circular DNA.

In addition to the time saving elements and simplification provided by the polynucleotide, the cell and method of the present invention, there may be further advantages provided by the use of the linear transcriptionally active polynucleotide because the amount of genetic material transfected into the cell is relatively small. The linear transcriptionally active polynucleotide may contain about 3,000 to 4,000 base pairs less than a plasmid comprising the same encoding sequence. The cells employed to express the linear transcriptionally active polynucleotide can readily accommodate a plurality of linear transcriptionally active polynucleotides. In fact multiple copies of the linear transcriptionally active polynucleotides are likely to be inserted into each cell. The cell is likely to be able to accommodate more copies of the linear transcriptionally active polynucleotides compared to the number of copies of the corresponding plasmids. Thus, the efficiency of expression per cell may be greater when the present method is employed compared to when a plasmid comprising the same encoding sequence is employed.

In the method according to the present invention, the cell may be transfected with one or more further linear transcriptionally active polynucleotides encoding distinct proteins. Accordingly, the cell may be transfected with two or more, three or more, four or more, five or more or ten or more distinct linear transcriptionally active polynucleotides.

In one embodiment the linear transcriptionally active polynucleotide is integrated into a chromosome or the genome of the cell to allow stable expression. In a further embodiment of the invention the cell is transiently transfected.

In the embodiment wherein a plurality of distinct linear transcriptionally active polynucleotides are employed the polynucleotides may be introduced into a cell simultaneously or sequentially. In one embodiment the linear transcriptionally active polynucleotides are transfected sequentially. If one polynucleotide encodes a polypeptide known to take longer time to be expressed compared to another polynucleotide, the transfection process may be initiated with only the slower expressing polynucleotide for a period such as 1, 2, 6, 12 or 18 hours followed by addition of the second polynucleotide after this initial period.

The cell according to the present invention may comprise a different quantity of distinct linear transcriptionally active polynucleotide. This embodiment is advantageous because it may improve the yield of expressed protein, particularly for multimeric proteins such as antibodies. The inventors have found that improved yield of antibody was achieved by transfecting the cell with more linear transcriptionally active polynucleotides encoding a light chain compared to the linear transcriptionally active polynucleotides encoding a heavy chain. Alternatively, the cell may comprise a higher quantity of linear transcriptionally active polynucleotides encoding a heavy chain compared to linear transcriptionally active polynucleotides encoding a light chain.

The linear transcriptionally active polynucleotide can be introduced into a cell using standard techniques, for example employing electroporation, or lipid-based methods (lipotransfection), anionic transfection, cationic transfection such as employing calcium phosphate, heat shock, magnetofection, transfection agents such as lipofectamine, dendrimers, DEAE-dextran transfection, transduction employing a virus. In one embodiment cationic transfection such as employing calcium phosphate is employed. Suitable insect transfection reagents include the Novagen Insect GeneJuice® Transfection Reagent, which is a liposome-based transfection reagent and Cellfectin® (Invitrogen).

When electroporation transfection is employed, the linear transcriptionally active polynucleotide for transfection can be added to a media comprising the relevant cells, before electroporation of the cells to facilitate the transfection process. Suitable conditions for electroporation may, for example, include 200-500 volts such as 250 volts; 1 or 2 pulses of 100 to 200 mircoseconds, with 0.1 to 5 seconds between pulses, performed in a 1mm electroporation curvette.

When lipotransfection is employed, the linear transcriptionally active polynucleotide for transfection will generally require pre-formulation to form a liposome before introduction to the cell. Thus, if a plurality of distinct polynucleotides are employed each polynucleotide may be separately formulated to form liposomes and optionally combined prior to transfection or a mixture of the linear transcriptionally active polynucleotides may be formulated into liposomes.

When cationic transfection is employed, a mixture of the linear transcriptionally active polynucleotide, the cells and the cationic agent can be introduced concomitantly.

The method according to the present invention may also employ a selection system to facilitate selection of stable cells which have been successfully transfected with the linear transcriptionally active polynucleotide. The selection system typically employs co-transfection of a polynucleotide sequence encoding a selection marker. In one embodiment, the linear transcriptionally active polynucleotide transfected into the cell further comprises a polynucleotide sequence encoding one or more selection markers. Accordingly, the transfection of the linear transcriptionally active polynucleotide and the one or more polynucleotides encoding the marker occurs together and the selection system can be employed to select those cells which produce the desired proteins.

Cells able to express the one or more markers are able to survive/grow/multiply under certain artificially imposed conditions. For example, a suitable toxin or antibiotic may be added to the growth medium accordingly to the properties endowed by the polypeptide/gene or polypeptide component of the selection system incorporated therein (e.g. antibiotic resistance). Those cells that cannot express the one or more markers are not able to survive/grow/multiply in the artificially imposed conditions. The artificially imposed conditions can be chosen to be more or less vigorous, as required.

Any suitable selection system may be employed in the present invention. Suitable selection systems include the use of geneticin, also known as G418, which is a toxin that can be neutralized by the product of a neomycin resistant gene; the use of the enzyme dihydrofolate reductase (DHFR), which is essential for the *de novo* synthesis of glycine, purine and thymidine, optionally in combination with an inhibitor of DHFR namely, methotrexate; and the use of glutamine synthetase (GS), which catalyses the formation of glutamine from glutamate and ammonia, optionally in combination with an inhibitor of GS, such as methionine sulphoximine (MSX). The zeocin selection system may also be employed in the present invention.

In one embodiment, the method according to the present invention further comprises the step of culturing the transfected cell in a medium to thereby express the fusion protein encoded by the linear transcriptionally active polynucleotide.

The skilled person would know from their common general knowledge suitable methods in the art to use to select suitable modified cell clones which express the proteins. For example, the skilled person may measure the mRNA expression of the protein from transfected cell clones and determine whether the level of mRNA expression of the protein is higher compared to unmodified cells. Techniques for measuring protein expression include ELISA, PCR analysis and Northern Blot analysis. The cell clones which exhibit expression of the desired protein may then be selected for further growth.

An inducible expression system may be used in the present invention to express the fusion protein encoded by the linear transcriptionally active polynucleotides. Suitable inducible expression systems are well known in the art.

Any suitable medium may be used to culture the transfected cell, for example Eagle media, fetal calf serum, cellgro or a proprietary media from a company such as Invitrogen. In some instances the media may be serum free. The medium may be adapted for a specific selection system, for example the medium may comprise an antibiotic, to allow only those cells which have been successfully transfected to grow in the medium. The method according to the present invention may also comprise a step of selecting those cells in the medium which have been successfully transfected, such as by selecting cells which are able to grow in the medium.

The cells obtained from the medium may be subjected to further screening and/or purification as required. The method may further comprise one or more steps to extract and purify the protein of interest as required.

One or more method steps described herein may be performed in combination in a suitable container such as a bioreactor.

Cell-free expression systems may be derived directly from a cell or may be a defined cell-free expression system not derived from a cell. Cell-free expression systems may be particularly advantageous for the expression of proteins from linear polynucleotides due to reduced adverse effects on the linear polynucleotides caused by cell expression. A cell-free expression system may also not require an agent to uncoil the linear polynucleotides as required for circular polynucleotide vectors. Suitable expression systems are known in the art, such as the Qiagen® EasyXpress® Insect Kit II which contains *Spodoptera frugiperda* insect cell extract, in vitro transcription reaction components, and reaction buffers (Szatmari, G., Hua, N.M., Vzdornov, D., Daigle, F., Smoragiewicz, W., Mamet-Bratley, M.D., Karska-Wysocki, B. (2006) In vitro expression of the restriction endonucleases LlaMI and ScrFI isolated from Lactococcus lactis M19 and UC503. J. Biotechnol. 121,144 and Lamla, T., Hoerer, S., and Bauer, M.M. (2006) Screening for soluble expression constructs using cell-free protein synthesis. Int. J. Biol. Macromol. 39, 111).

The present invention also provides an expression system comprising the linear transcriptionally active recombinant polynucleotide, as defined above, and a solvent or medium. The expression system may further comprise one or more further distinct linear transcriptionally active recombinant polynucleotides, as defined above. The expression system may be suitable for cell-dependent expression. In a preferred embodiment, the expression system comprises a host cell as defined above and a solvent or medium.

Any suitable solvent or medium may be used in the expression system including water or tris-based buffer for the polynucleotide and a suitable growth media such as CD-CHO FreeStyle™ media for the host cell.

The transcriptionally active recombinant linear polynucleotides employed in the present invention may be made by any suitable method.

The transcriptionally active recombinant linear polynucleotides can be prepared employing PCR, for example using suitable oligonucleotide primers. Nucleic acid amplification methods are well known in the art. Where the nucleic acid which has been recovered is RNA, the RNA may be reverse transcribed to give cDNA. In addition to PCR, other amplification procedures may be used. Other amplification procedures include the T7 and Q-replicase methods.

The linear transcriptionally active polynucleotides can be prepared employing PCR, for example using suitable oligonucleotide primers. Nucleic acid amplification methods are well known in the art. Where the nucleic acid which has been recovered is RNA, the RNA may be reverse transcribed to give cDNA. In addition to PCR, other amplification procedures may be used. Other amplification procedures include the T7 and Q-replicase methods.

In the embodiment wherein the linear transcriptionally active polynucleotides encode a light chain and/or a heavy chain of an antibody, typcially PCR will be employed to provide each polynucleotide comprising attaching the variable domain from the heavy chain or light chain identified, for example in a previous screen, to a suitable promoter at the 5' end and optionally an entity at the 3' end comprising at least a polyadenylation sequence and/or a constant domain, such that a sequence encoding a full or partial heavy chain or light chain may be formed.

Preferably, the linear transcriptionally active polynucleotides are made by the method as described in US 6,280,977 and Liang, X et al, 2002, The Journal of Biological Chemistry, 227(5), 3593-3598. These documents describe TAP fragments produced by PCR amplification of a DNA molecule in the presence of a first DNA fragment comprising a promoter sequence; a second DNA fragment comprising a terminator sequence; a first primer complementary to the 5' end of the first DNA fragment; a second primer complementary to the 3' end of the second DNA fragment; a third primer comprising a first region complementary to the 3' end of the first DNA fragment and a second region complementary to the 5' end of the DNA molecule; and a fourth primer comprising a first region complementary to the 5' end of the second DNA fragment and a second region complementary to the 3' end of the DNA molecule.

PCR amplification may be employed to produce the linear transcriptionally active polynucleotides employed in the present invention, in the embodiment where each linear transcriptionally active polynucleotide comprises a sequence comprising an encoding polynucleotide sequence (F2), a sequence comprising a first transcription regulatory element (F1) at the 5' end of the sequence comprising the encoding polynucleotide sequence (F2) and a 3' end sequence (F3) at the 3' end of the sequence comprising the encoding polynucleotide sequence (F2). In the present invention the 3' end polynucleotide sequence (F3) may comprise a second transcription regulatory element and/or a second encoding polynucleotide sequence, as discussed previously.

It is important that the primers are designed appropriately to ensure the success of the PCR amplification method. Typically, two pairs of primers are employed in the method. A pair of "internal primers" (discussed below as the first primer P1 and second primer P2), wherein the first primer (P1) comprises a region complementary to the 5' end of the sequence comprising the encoding polynucleotide sequence (F2) and an overlap-extension tail complementary to the 3' end of the sequence comprising the first transcription regulatory element (F1) that will be attached thereto. The second internal primer (P2) comprises a region complementary to the 3' end of the sequence comprising the encoding polynucleotide sequence (F2) and an overlap-extension tail complementary to the 5' end of the 3' end polynucleotide sequence (F3) that will be attached thereto. The overlap extension tails in the first (P1) and second primers (P2) ensures the joining of the sequence comprising the first transcription regulatory element (F1), the sequence comprising the encoding polynucleotide sequence (F2) and the 3' end polynucleotide sequence (F3). A pair of "external" primers (discussed below as the third primer (P3) and fourth primer (P4)), which in relation to the overall linear transcriptionally active polynucleotide sequence assembled are located at the extremities i.e. the absolute 3' end and the 5' end of the sequence respectively.

As shown in Figure 2a, in a first PCR step (PCR1) the method comprises PCR amplification of the sequence comprising the coding polynucleotide sequence (F2) in the presence of the first primer (P1) and the second primer (P2) thereby producing an intermediate PCR product comprising the encoding polynucleotide sequence (F2), the 3' end of the sequence comprising the first transcription regulatory element (F1) at the 5' end of the sequence comprising the encoding polynucleotide sequence (F2) and the 5' end of the 3' end polynucleotide sequence (F3) at the 3' end of the sequence comprising the encoding polynucleotide sequence (F2).

As show in Figure 2a, in a second PCR step (PCR2) the method comprises PCR amplification of the intermediate PCR product in the presence of the sequence comprising the first transcription regulatory element (F1), the 3' end polynucleotide sequence (F3), the third primer (P3) and the fourth primer (P4) thereby producing a transcriptionally active recombinant linear polynucleotide comprising the first transcription regulatory element (F1) and the encoding polynucleotide sequence (F2) and the 3'end polynucleotide sequence (F3).

The first and second PCR steps may be carried out separately or simultaneously.

In the embodiment wherein each transcriptionally active polynucleotide sequences comprises a plurality of encoding polynucleotide sequences, the encoding polynucleotide sequences may have been already linked together and the above PCR method is carried out to fuse the encoding polynucleotide sequences to the first transcription regulatory element (F1) and 3'end polynucleotide sequence (F3). Alternatively, the encoding polynucleotide sequences may be linked together via overlap extension PCR using the method as described below.

The present invention also provides a method of producing a transcriptionally active recombinant linear polynucleotide comprising a plurality of encoding polynucleotide sequences and one or more transcription regulatory elements, wherein each encoding polynucleotide sequence encodes a subunit of a fusion protein, wherein the method comprises:
a) providing a first encoding polynucleotide sequence, a second encoding polynucleotide sequence and one or more transcription regulatory elements;
b) fusing the first encoding polynucleotide sequence and the second encoding polynucleotide sequence, and
c) fusing one or more transcription regulatory elements to the first encoding polynucleotide sequence and/or the second encoding polynucleotide sequence.

Step b) and step c) according to the above method may be carried out simultaneously or sequentially in any order. In one embodiment step b) and/or step c) is effected employing PCR.

Step a) may comprise providing one or more further encoding polynucleotide sequences and step b) may therefore comprise fusing the one or more further encoding polynucleotide sequences to the first and second encoding polynucleotide sequences.

Preferably step a) comprises PCR amplification of a sequence comprising the first encoding polynucleotide sequence in the presence of:
a first primer comprising a region complementary to the 5' end of the sequence comprising the first encoding polynucleotide sequence and an overlap-extension tail complementary to the 3' end of a sequence comprising a first transcription regulatory element; and
a second primer comprising a region complementary to the 3' end of the sequence comprising the first encoding polynucleotide sequence and an overlap-extension tail complementary to the 5' end of a second comprising the second encoding polynucleotide sequence;
thereby producing an intermediate PCR product comprising the first encoding polynucleotide sequence, the 3' end of the sequence comprising the first transcription regulatory element at the 5' end of the sequence comprising the first encoding polynucleotide sequence and the 5' end of the sequence comprising the second encoding polynucleotide sequence at the 3' end of the sequence comprising the first encoding polynucleotide sequence.

Preferably step b) and step c) are carried out simultaneously by PCR amplification of the intermediate PCR product in the presence of a sequence comprising the first transcription regulatory element, a sequence comprising the second encoding polynucleotide sequence, a third primer complementary to the 5' end of the sequence comprising the first transcription regulatory element and a fourth primer complementary to 3' end of the sequence comprising the second encoding polynucleotide thereby producing a transcriptionally active recombinant linear polynucleotide comprising the first transcription regulatory element, the first encoding polynucleotide sequence and the second encoding polynucleotide sequence.

In a preferred embodiment the sequence comprising the second encoding polynucleotide sequence comprises a second transcription regulatory element at the 3' end of the second encoding polynucleotide sequence, preferably a polyadenylation sequence. It is also preferred that the first transcription regulatory element comprises a promoter at the 5' end of the first encoding polynucleotide sequence.

The primers are the same as those discussed above.

Accordingly, in the embodiment where each linear transcriptionally active polynucleotide comprises a sequence comprising a first encoding polynucleotide sequence (F2), a sequence comprising a first transcription regulatory element (F1) at the 5' end of the sequence comprising the encoding polynucleotide sequence (F2) and a sequence comprising a second encoding polynucleotide (F3) at the 3' end of the sequence comprising the first encoding polynucleotide sequence (F2), as shown in Figure 2b, step a) preferably comprises a first PCR amplification step (PCR1) of the sequence comprising the first coding polynucleotide sequence (F2) in the presence of the first primer (P1) and the second primer (P2) thereby producing an intermediate PCR product comprising the first encoding polynucleotide sequence (F2), the 3' end of the sequence comprising the first transcription regulatory element (F1) at the 5' end of the sequence comprising the first encoding polynucleotide sequence (F2) and the 5' end of the sequence comprising the second encoding polynucleotide sequence (F3) at the 3' end of the sequence comprising the first encoding polynucleotide sequence (F2).

The use of Taq DNA polymerase may add an extra adenosine base (A) at the 3' end of the PCR fragment produced. In this situation, there may be a mismatch between the intermediate PCR product produced by the first PCR step and the first transcription regulatory element (F1) and the 3'end polynucleotide sequence (F3). Accordingly, in one embodiment of the present invention the first transcription regulatory element (F1) and the 3' end polynucleotide sequence (F3) are provided with a thymidine base (T) immediately preceding the overlap complementary region with the intermediate PCR product. Accordingly, if adenosine bases are added to the 3' ends of the intermediate PCR product, the intermediate PCR product will still be complementary to the end of the first transcription regulatory element (F1) and the end of the 3'end polynucleotide sequence (F3).

As show in Figure 2b, steps b) and c) may be carried out simultaneously in a second PCR amplification step (PCR2) of the intermediate PCR product in the presence of the sequence comprising the first transcription regulatory element (F1), the second encoding polynucleotide sequence (F3), the third primer (P3) and the fourth primer (P4) thereby producing a transcriptionally active recombinant linear polynucleotide comprising the first transcription regulatory element (F1) and the encoding polynucleotide sequence (F2) and the second encoding polynucleotide sequence (F3).

The first and second PCR steps may be carried out separately or simultaneously.

In one embodiment, after the first PCR step has produced a sufficient quantity of the intermediate PCR product, an agent may be added to the PCR reaction mixture which stops the PCR amplification of PCR 1 with primers P1 and P2. An example of such an agent is ExoSap-IT® (USB), which acts to degrade single stranded oligonucleotides including the primers P1 and P2 thereby ensuring that PCR1 amplification does not continue in PCR2 amplification with primers P3 and P4. An additional or alternative means to prevent PCR1 amplification with primers P1 and P2 to continue during PCR2 amplification with primers P3 and P4 is to limit the quantity of primers P1 and P2 used in PCR1 to a suitable quantity required to produce sufficient quantity of the intermediate PCR product during PCR1. A suitable quantity of primers P1 and P2 to produce sufficient quantity of the intermediate PCR1 may be determined by titration methods known in the art.

One or more of the polynucleotide fragments F1, F2 and F3 may comprise one or more non-coding/non-functional nucleotide sequences (i.e. sequences which do not encode polypeptide sequences or function as a transcription regulatory element). Accordingly, if one or more of the polynucleotide fragments F1, F2 and F3 comprises non-coding/non-functional nucleotides at the 5' and/or 3 end, one or more of the primers PI, P2, P3 and P4 may comprises sequences which are complementary for the non-coding/non-functional sequence of the fragment or may comprise sequences which are partly complementary for the non-coding/non-functional sequence of the fragment and partly complementary to the transcription regulatory element or encoding polynucleotide sequence contained in the fragment. For example, F1 may comprise a non-coding/non-functional nucleotide sequence at the 3' end of F1 and, therefore, the primer P1 may comprise an overlap extension tail complementary to the non-coding/non-functional region at the 3'end of F1. A further example is where the F2 may comprise a non-coding/non-functional nucleotide sequence at the 5' end of the first encoding polynucleotide sequence, such as a leader sequence, and, therefore, the primer P1 may comprise a region complementary to the 5' non-coding/non-functional sequence of F2. A further example is where F1 may comprise a non-coding/non-functional sequence at the 5' end and, therefore P3 primer may be complementary to the non-coding/non-functional sequence at the 5' end of F1. A further example is where F3 may comprise a non-coding/non-functional sequence at the 3' end and, therefore P4 primer may be complementary to the non-coding/non-functional sequence at the 3' end of F3.

If the polynucleotide fragments do not comprise one or more non-coding/non-functional nucleotide sequence the primers PI, P2, P3 and P4 may comprise sequences which are fully complementary for the transcription regulatory element or encoding polynucleotide sequence contained in the respective fragment.

The one or more final linear transcriptionally active polynucleotide produced may be analyzed by any suitable method, such as agarose gel electrophoresis, for confirmation that the PCR product generated is the expected length.

In the embodiment wherein the first encoding polynucleotide sequence encodes a variable domain or fragment thereof of an antibody and the second encoding polynucleotide sequence encodes one or more constant domains or fragment thereof of an antibody the method described above may also be employed to produce the linear transcriptionally active polynucleotide. In the embodiment wherein a linear transcriptionally active polynucleotide encodes a light chain and a linear transcriptionally active polynucleotide encodes a heavy chain, each linear transcriptionally active polynucleotide may be produced using the above described PCR method. The first PCR step for the light chain encoding sequence and the heavy chain encoding sequence may be carried out simultaneously (i.e. in a multiplex step) or separately (i.e. not multiplex). The second PCR step for the light chain encoding sequence and the heavy chain encoding sequence may be carried out simultaneously (i.e. in a multiplex step) or separately (i.e. not multiplex).

Each linear transcriptionally active polynucleotide preferably comprises 5' promoter sequence, a sequence encoding the antibody light chain sequence or fragment thereof or heavy chain sequence or fragment thereof, and a 3' polyadenylation sequence. Each linear transcriptionally active polynucleotide preferably comprises 5' promoter sequence, a first encoding sequence encoding the heavy chain or light chain variable domain sequence or fragment thereof (VH or VL), a second encoding sequence encoding the one or more heavy chain domain sequences (CHI, CH2, CH3) or light chain constant domain sequences (CL) or fragment thereof and a 3' polyadenylation sequence.

The generation of either a light chain linear transcriptionally active polynucleotide or a heavy chain linear transcriptionally active polynucleotide follows the same PCR steps as described above, wherein:
▪ the first transcription regulatory element (F1) is a sequence comprising the 5' promoter;
▪ the first encoding polynucleotide sequence (F2) comprises the antibody variable domain sequence (VH or VL); and
▪ the second encoding polynucleotide sequence (F3) comprises a polyadenylation sequence optionally together with the one or more antibody constant domain sequences (CH or CL) positioned upstream of the polyA sequence.

In the first PCR step the antibody variable domain sequence (F2) is amplified in the presence of the first primer (P1) and the second primer (P2) to generate the intermediate PCR product comprising the variable domain sequence (F2) having a 5' end sequence complementary to the 3' end of the promoter sequence (F1) and a 3' end sequence complementary to the 5' end of the 3' end polynucleotide sequence (F3). The design of primers P1 and P2 for the first PCR step relies on knowledge of the antibody variable domain encoding sequence (F2). Primers P1 and P2 can be designed for each separate light chain and heavy chain variable domain encoding sequence (F2).

In the second PCR step the intermediate PCR product generated in the first PCR step is amplified in the presence of the 5' promoter sequence (F1), the 3' end polynucleotide sequence (F3), primer P3 which is an external primer complementary to the 5' end of the 5' promoter sequence (F1) and primer P4 which is an external primer complementary to the 3' end of the 3' end polynucleotide sequence (F3). The second PCR step generates the complete linear transcriptionally active polynucleotide comprising the 5' promoter sequence (F1), the light chain or heavy chain variable domain encoding sequence (F2) and the polyadenylation sequence optionally together with the antibody constant domain sequence (F3).

In one embodiment of the present invention, when the F3 sequence comprises a constant domain sequence, a number of different antibodies may be easily produced by varying the constant domain sequence of F3. Accordingly, different antibodies may be produced quickly and easily using the above described method. The constant domains may be derived from different isotypes and/or species, such as rabbit or mouse. The F3 sequence may also be easily varied to provide different fragments of antibodies. For example, the F3 sequence may comprise the CH1 constant domain to provide a Fab antibody or the F3 may comprise CH1-CH2-CH3 to provide a full length antibody. Therefore, the linear transcriptionally active polynucleotides employed in the present invention allow a diverse range of antibodies to be easily produced and expressed for screening purposes.

The method may be repeated to produce a light chain transcriptionally active recombinant linear polynucleotide and a heavy chain transcriptionally active recombinant linear polynucleotide. The method may also comprise a further step of fusing the transcriptionally active recombinant linear polynucleotide encoding the heavy chain and transcriptionally active recombinant linear polynucleotide encoding the light chain.

In one embodiment the present invention provides one or more primer sequences selected from SEQ ID NO:1, SEQ ID NO:3, SEQ ID NO:5, SEQ ID NO:7 and SEQ ID NO:9. These primer sequences may be used to generate one or more transcriptionally active recombinant linear polynucleotides by the method described above. The primer sequences are described in detail above and in the examples.

The one or more transcription regulatory elements and encoding polynucleotide sequences employed to produce the linear transcriptionally active polynucleotide may be generated by any suitable means. The transcription regulatory elements, such as the promoter and polyadenylation sequences, may be generated by restriction digest of a previously prepared plasmid DNA construct.

The encoding polynucleotide sequences may be generated by any suitable method including PCR amplification.

In one embodiment the method according to the present invention is performed after identification of an antibody of interest (or a fragment thereof), such as by phage display technology.

In the embodiment wherein one or more of the encoding polynucleotide sequences comprise variable domains of an antibody, the sequences may be generated by PCR amplification of antibody-encoding nucleic acid sequences generated by SLAM (selected lymphocyte antibody method) as set forth in WO92/02551. Accordingly, in one embodiment, the method according to the present invention is employed as further step in the SLAM process which enables a single lymphocyte that is producing an antibody with a desired specificity to be identified within a large population of lymphoid cells and the genetic information that encodes the specificity of the antibody (the variable domains or regions) to be identified from that lymphocyte (Babcook et al., 1996, Proc. Natl. Acad. Sci, 93, 7843-7848).

In the SLAM process a population of antibody-forming cells is cultured in a medium having an indicator system incorporated therein which is capable of indicating the presence and location of a cell which forms antibodies exhibiting the desired function. This allows a cell to be identified from the medium which forms the desired antibodies and the amino acid sequence of the variable domain which confers the desired function of the antibody can be determined. In the SLAM process the variable domain sequence information is then used to synthesize a protein with a desired function usually by incorporating the DNA sequence corresponding to the amino acid sequence of the variable domain into a vector capable of directing the expression and secretion of a protein with the desired function. This final cloning step is a low throughput process in comparison to the previous steps of the SLAM process which are high throughput and capable of automation. However, the cell and method according to the present invention allow a faster and simplified means to produce antibodies or fragments thereof incorporating the variable domain sequence identified by the SLAM process. Accordingly, the present invention allows the final stage of the SLAM process to be high throughput, thus allowing more antibodies to be produced more quickly incorporating various variable domain sequences identified as conferring the desired activity on the antibodies.

Accordingly, the present invention also provides a method for obtaining a recombinant antibody with a desired function, comprising:
(a) providing a population of antibody-forming cells suspected of containing at least one cell capable of producing an antibody exhibiting the desired function;
(b) generating one or more transcriptionally active recombinant linear polynucleotides from the antibody forming cells obtained in step (a) wherein each transcriptionally active recombinant linear polynucleotide comprises a polynucleotide sequence encoding a variable domain of an antibody produced by an antibody-forming cell obtained in step (a) and one or more transcription regulatory elements;
(c) expressing a recombinant antibody using one or more of the transcriptionally active recombinant linear polynucleotides generated in step (b);
(d) screening the recombinant antibody produced by step (c) for the desired function; and
(e) optionally repeating steps (b), (c) and (d) to identify a recombinant antibody exhibiting the desired function.

The use of one or more transcriptionally active recombinant linear polynucleotides to express a recombinant antibody comprising a variable domain of an antibody produced by an antibody forming cell in the method provides many significant improvements to a method for producing a recombinant antibody. The use of one or more transcriptionally active recombinant linear polynucleotides provides a far quicker means for cloning variable domain genes and expressing recombinant antibodies compared to the multiple digestion and ligation steps required to produce vectors and allow a faster and easier way to obtain the recombinant protein.

The population of antibody forming cells provided in step (a) may be from any suitable source. Typically, the population of antibody-forming cells is obtained from an animal which has been immunized with a selected antigen or obtained from an animal which generated said cells during the course of a selected disease. The antibody forming cells are typically B-lymphocytes or its progeny including plasma cells. The cells may either secrete antibodies or maintain antibodies on the surface of the cell without secretion into the cellular environment. The antibody-forming cells may be obtained from blood or directly from bone marrow and/or other lymphoid tissue. Further detail regarding the type and origin of antibody-forming cells may be found in WO 92/02551.

Step (b) requires the generation of one or more transcriptionally active recombinant linear polynucleotides each comprising a polynucleotide sequence encoding a variable domain of an antibody produced by an antibody-forming cell. The transcriptionally active recombinant linear polynucleotide and methods of generating a transcriptionally active recombinant linear polynucleotide have been described in detail above.

The variable domain of an antibody produced by an antibody-forming cell may be incorporated into a transcriptionally active recombinant linear polynucleotide using any suitable method known in the art. In one embodiment the sequence of the variable domain or one or more fragments thereof which confer the specificity of an antibody produced by an antibody-forming cell may be determined. The use of one or more linear polynucleotides to express the recombinant antibody allows the sequence of the variable domain to be determined during the generation of the one or more transcriptionally active linear polynucleotides in step (b) after any PCR step. However, the sequence of the variable domain or one or more fragments thereof which confer the specificity of the antibody does not need to be determined in order to generate the one or more transcriptionally active recombinant linear polynucleotides. Accordingly, in one embodiment the method does not comprise a step of sequencing the variable domain of the antibody produced by the antibody-forming cell prior to and/or during step (b). Specially designed primers which are complementary to each end of the variable domain or one or more fragments thereof of the antibody, may be used to clone the variable domain or one or more fragments thereof by PCR. The primers may be complementary to the framework regions either side of one or more CDRs of the antibody. Further detail regarding how the variable domain or a portion thereof of an antibody produced by an antibody-forming cell may be determined may be found in WO 92/02551.

In step (b) the variable domain of an antibody produced by an antibody-forming cell which is included in the transcriptionally active recombinant linear polynucleotide may be the whole variable domain of the antibody or one or more fragments thereof which confer the specificity of the antibody including one, two or three CDR regions of a variable domain. Each transcriptionally active recombinant linear polynucleotide encodes a variable domain or a fragment thereof of an antibody produced by an antibody-forming cell. The variable domain or fragment thereof is the minimum amount of sequence derived from an antibody produced by an antibody-forming cell that each transcriptionally active recombinant linear polynucleotide contains. However, each transcriptionally active recombinant linear polynucleotide may comprise one or more further antibody domains ether derived from an antibody produced by an antibody-forming cell or from a different source. As described above, each transcriptionally active recombinant linear polynucleotide may comprise a sequence encoding a fusion protein of a variable domain and one or more constant domains.

The antibody produced by the antibody-forming cell may be a single-domain antibody such as a VHH. In one embodiment the antibody produced by the antibody-forming cell comprises a heavy chain and a light chain.

The light chain variable domain or one or more fragments thereof and the heavy chain variable domain or one or more fragments thereof from the antibody produced by the antibody-forming cell are preferably incorporated into the one or more transcriptionally active recombinant linear polynucleotides in step (b). In this embodiment, step (b) may comprise generating two distinct transcriptionally active recombinant polynucleotides for each recombinant antibody: a transcriptionally active recombinant linear polynucleotide encoding a light chain or a fragment thereof and a transcriptionally active recombinant linear polynucleotide encoding a heavy chain or a fragment thereof for each recombinant antibody. Alternatively, or additionally step (b) may comprise generating a transcriptionally active recombinant linear polynucleotide encoding both a light chain or fragment thereof and a heavy chain or fragment thereof for each recombinant antibody. As discussed previously, the light chain or a fragment thereof is preferably encoded by a variable domain encoding polynucleotide sequence and a constant domain encoding polynucleotide sequence and the heavy chain or a fragment thereof is preferably encoded by a variable domain encoding polynucleotide sequence and one or more constant domain encoding polynucleotide sequences.

In one embodiment the recombinant antibody encoded by the one or more transcriptionally active recombinant linear polynucleotides comprises variable domains or one or more CDRs thereof derived from an antibody produced by an antibody-forming cell and further comprises variable domain framework regions and/or one or more constant domains derived from a different source, such as a known human antibody sequence in order to provide a chimeric or humanized recombinant antibody. The use of transcriptionally active recombinant linear polynucleotide allows one or more variable domain derived from an antibody produced by an antibody-forming cell to be incorporated into any suitable recombinant antibody format, such as a Fab or a full-length antibody with varying constant regions, easily and efficiently.

In this embodiment wherein the antibody comprises a heavy chain and a light chain, the recombinant antibody expressed in step (c) preferably retains the original light chain and heavy chain pairing of an antibody produced by an antibody-forming cell. However, in one embodiment, wherein a plurality of antibody-forming cells are taken forward together in step (b) the recombinant antibodies expressed in step (c) may or may not retain the original light and heavy chain pairing of an antibody produced by an antibody-forming cell.

Step (c) comprises expressing a recombinant antibody using one or more of the transcriptionally active recombinant linear polynucleotides generated by step (b). Far higher concentrations of a recombinant antibody can be produced from step (c) of the method of the present invention compared to the quantity of antibody being produced in a population of antibody-forming cells, such as B-lymphocytes obtained from an animal. In one embodiment step (c) comprises transfecting the transcriptionally active recombinant linear polynucleotide into a host cell; and growing said host cells in an appropriate medium as described above. Alternatively, or additionally, step (c) comprises expressing the antibody from the transcriptionally active recombinant linear polynucleotide in a cell-free expression system as described above. The use of a cell-free expression system provides a further means for reducing the number of steps required to produce the recombinant protein because steps such as cell transfection are not required for cell-free expression. Accordingly, protein may be produced more quickly and efficiently.

Step (d) comprises screening the recombinant antibody produced by step (c) for the desired function. The screening may be performed by any suitable method known in the art to test the function of an antibody. Functions of the antibody which may be tested include the ability to bind a selected antigen and/or the affinity of the antibody for a selected antigen and/or ability to function in a particular assay e.g. neutralization, agonise or antagonize a given biological activity. It will be appreciated that more than one screen may be performed and more than one function tested e.g. binding and functional activity.

Step (d) preferably comprises screening to detect binding of the recombinant antibody to a selected antigen. The selected antigen may be a purified antigen or a cell surface expressed antigen. The selected antigen may or may not be known. Accordingly, the screening step in (d) may include detecting a function of the antibody which does not require knowledge of the antigen such as the effect of the recombinant antibody on the modification of a substrate or the effect on the growth, viability or function of a layer of cells or the effect on the pathogenicity of a pathogenic microorganism. Suitable screening tests are described in detail as indicator systems in WO92/02551.

In one embodiment the method comprises a first screening step prior to step (b) in which the population of antibody-forming cells provided in step (a) are screened to identify an antibody-forming cell or a population of antibody-forming cells producing an antibody exhibiting the desired function. As described above for step (d), the first screening step may include detecting binding to a selected antigen and/or affinity of the antibody to a selected antigen. The identity of the antigen may or may not be known in the first screening step. Accordingly, the first screening step may not require knowledge of the selected antigen. The first screening step may include detecting a function of the antibody which does not require knowledge of the antigen such as the effect of the antibody on the modification of a substrate or the effect on the growth, viability or function of a layer of cells or the effect on the pathogenicity of a pathogenic microorganism. In this embodiment the first screening step identifies a cell or a population of cells and in step (b) the one or more transcriptionally active recombinant linear polynucleotides are generated from the identified cell or population of cells. The first screening step may identify an individual antibody-forming cell or a population of antibody-forming cells which produce an antibody exhibiting the desired function. It will be appreciated that the antibody-forming cell or cells which produce an antibody exhibiting the desired function may be within a population of other cells which do not produce antibodies exhibiting the desired function i.e. it is not necessary for all cells in a population identified by the first screening step to be producing antibody exhibiting the desired function.

In this embodiment the method preferably further comprises an isolating step prior to step (b) of isolating a single antibody-forming cell producing an antibody exhibiting the desired function identified by the first screening step and in step (b) the one or more transcriptionally active recombinant linear polynucleotides are generated from the single isolated antibody-forming cell. The single antibody-forming cell may be isolated from a population of antibody-forming cells identified by the first screening step. The inclusion of an isolating step is particularly advantageous when the antibody produced by the antibody-forming cell comprises a heavy chain and a light chain because it allows a single recombinant antibody to be expressed which retains the original heavy chain and light chain variable domain pairing of the antibody produced by the antibody-forming cell.

The isolating step may be carried out by any suitable means. In one embodiment the first screening step and the isolating step are carried out simultaneously. In a further embodiment, the first screening step is first carried out to identify an antibody-forming cell or a population of antibody forming cells producing an antibody exhibiting the desired function and the isolating step is subsequently carried out. In this embodiment, the isolating step may comprise a second screening step to identify an antibody-forming cell producing an antibody exhibiting the desired function. Alternatively, the isolating step comprises isolating a single antibody-forming cell without a second screening step to identify an antibody-forming cell producing an antibody exhibiting the desired function. The single antibody-forming cell may be isolated from a population of antibody-forming cells identified from the first screening step.

An example of a suitable method for carrying out the isolating step, which preferably allows simultaneous screening and isolating, is described in WO 92/02551. The method described in WO 92/02551 comprises
i) suspending the population of antibody-forming cells obtained in step (a) in a medium, the medium having an indicator system incorporated therein, said indicator system being capable of indicating the presence and location of a cell which forms antibodies exhibiting the desired function;
ii) identifying a single cell forming an antibody exhibiting the desired function; and
iii) isolating the identified single antibody-forming cell from the medium.

As described in WO 92/02551 the indicator system manifests a specific desired activity in response to antibody on the surface of or released into the vicinity of an antibody-forming cell, thus permitting identification and isolation of the cell which produces the antibody with the desired function. As described above, the indication of antibodies exhibiting the desired function in step i) may comprise detecting binding to a selected antigen and/or affinity of the antibody to a selected antigen. The identity of the antigen may or may not be known in step i). Step i) may include detecting a function of the antibody which does not require knowledge of the antigen.

As described in WO 92/02551 the indicator system in step i) may comprise:
- a layer of cells whose growth, viability, or function is affected by antibodies exhibiting a desired function produced by the isolated antibody-forming cell;
- one or more pathogenic microorganisms, and a layer of cells susceptible to infection by said microorganisms, wherein said antibodies exhibiting a desired function are identified as those which effect the pathogenicity of the microorganism;
- a set of two cell types selected from the group consisting of cells of distinct HLA histocompatibility antigen types, blood group antigen types, and tumor cells and normal cells of the same lineage, wherein said antibodies exhibiting a desired function are identified as those which agglutinate or lyse one cell of the pair;
- erythrocytes or other particles coated with antigen, and said antibody exhibiting the desired function is identified as that which binds to the antigen, thereby causing the particles to agglutinate;
- erythrocytes or other particles coated with antigen, and said antibody exhibiting the desired function is identified as that which binds to the antigen, lysing the cells or particles in the presence of complement;
- a complexing factor and said antibody-forming cell is identified as that which binds to the complexing factor, forming a rosette; or
- a substrate, and said antibody exhibiting a desired function is identified as that which modifies the substrate in a detectable manner.

In one embodiment step i) may comprise the method described in WO 2004/051268 and WO 2005/121789 of incubating said population of antibody producing cells obtained in step (a) with a selected antigen and a labeled anti-antibody antibody, wherein said anti-antibody antibody is capable of distinguishing cells producing an antibody which binds to the selected antigen from those cells which do not; and step ii) comprises identifying a single antibody-forming cell capable of producing an antibody which binds to the selected antigen. As described in WO 2004/051268 and WO 2005/121789, the labeled anti-antibody antibody is preferably an anti-Fc antibody. The anti-antibody antibody is preferably labeled with a fluorescent conjugate.

A further example of a suitable method for carrying out the isolating step, which preferably allows simultaneous screening and isolating, is described in WO 2004/106377. The method described in WO 2004/106377 comprises
i) bringing the population of antibody-forming cells obtained in step (a) into contact with a capturing agent;
ii) separating the captured antibody-forming cells from the uncaptured antibody-forming cells;
iii) culturing a plurality of captured antibody-forming cells wherein the antibody-forming cells have not been sorted into single antibody-forming cells immediately prior to culturing; and
iv) screening a plurality of the cultured antibody-forming cells to identify a single cell forming an antibody exhibiting the desired function; and
v) isolating the identified single antibody-forming cell.

As described in WO 2004/106377, the separating step is preferably panning and the captured cells are cultured directly following panning. The capturing agent is preferably an antigen.

The step of isolating a single antibody-forming cell may also be carried out by single cell sorting flow cytometry. The single cell sorting flow cytometry may be used to isolate a single antibody-forming cell using any suitable measurable parameters. For example, flow cytometry may be used to isolate a single antibody-forming cell having an antibody-forming cell marker, such as a B-cell marker. Flow cytometry may be used to isolate a single antibody-forming cell capable of binding to a selected antigen. Suitable methods of cell sorting which may be used in the method of the present invention, specifically in the step of isolating, are described in WO05/019824 and WO05/019823.

A further example of means to isolate a single antibody-forming cell is by automated microscopy and manipulation such as laser capture microscopy.

In one embodiment, prior to step (b) the method comprises an isolating step of directly isolating a single antibody-forming cell from the population of antibody-forming cells obtained in step (a) without first screening the population of antibody-forming cells provided in step (a) for an antibody-forming cell producing an antibody exhibiting the desired function and in step (b) the one or more transcriptionally active recombinant linear polynucleotides are generated from the single isolated antibody-forming cell.

In this embodiment the method does not comprise a separate screening step prior to the isolating step, which is referred to above as the first screening step. However, the step of isolating may itself comprise a simultaneous screening step to identify an antibody-forming cell producing an antibody exhibiting the desired function, which is referred to above as the second screening step. Alternatively, the isolating step comprises isolating a single antibody-forming cell without a separate prior screening step or a simultaneous screening step to identify an antibody-forming cell producing an antibody exhibiting the desired function. Suitable means for isolating a single antibody-forming cell have been described in detail above, including the method described in WO 92/02551, the method described in WO 2004/051268 and WO 2005/121789, the method described in WO 2004/106377, single cell sorting flow cytometry or automated microscopy and manipulation.

In this embodiment the method does not comprise a first screening step prior to generation of the one or more transcriptionally active recombinant linear polynucleotides. Accordingly, the speed and efficiency of the method is further increased by eliminating early screening step(s). The use of one or more transcriptionally active recombinant linear polynucleotides allows the early screening step(s) of the antibody-forming cells to be eliminated because it allows high throughput expression of the recombinant antibody. Screening is performed after generation of the recombinant antibody in step (d). A number of advantages result from screening the recombinant antibody for the desired function in step (d) after using one or more transcriptionally active recombinant linear polynucleotides to express the recombinant antibody compared to screening the population of antibody-forming cells for desired function. One advantage is that there is less interference from unknown factors in the recombinant antibody expression media produced in step (c) compared to the media comprising the population of antibody-forming cells. Further, performing screening on the population of antibody producing cells obtained in step (a) may result in antibodies having the desired function being disregarded incorrectly. However, the method of the present invention allows a greater number and variety of recombinant antibodies produced in step (c) to be screened for the desired function as all are expressed at high levels.

Suitable pharmaceutically acceptable carriers including solvents, solubilizers, fillers, stabilizers and the like are well known in the art. The pharmaceutical composition may be formulated for any intended route of administration including but not limited to parental, intravenous, oral, inhalation, topical and systemic administration. In one embodiment the linear transcriptionally active recombinant polynucleotide as defined above and/or a cell as defined above are suitable for gene delivery for being introduced into a patient. The linear transcriptionally active recombinant polynucleotide may be encapsulated in a liposome for gene delivery. Various other methods of introducing genes into a patient are well known in the art.

The encoding polynucleotide sequence in the linear transcriptionally active polynucleotides may encode a therapeutic or prophylactic fusion protein suitable for the treatment of a human or non-human animal in need thereof. Accordingly, the protein produced by the method according to the present invention, such as an antibody, may be used for the treatment of disease such as HIV; malaria; allergy; HCV; autoimmune diseases such as irritable bowel syndrome, rheumatoid arthritis and multiple sclerosis; cancer in particular breast cancer, lung cancer, pancreatic cancer, colon cancer, liver cancer, head and neck cancer, leukemia, myeloma and the like.

One or more embodiments of the invention described herein may be combined unless they are technically incompatible.

### Examples

### Example 1 - The first PCR step (PCR1) in the generation of linear transcriptionally active polynucleotides.

### Primers for PCR 1

Primers were designed suitable for a first PCR amplification (PCR1) of a variable heavy chain encoding sequence (F2 VH) and a variable light chain encoding sequence (F2 VL) of an anti-human soluble cytokine mouse antibody of known sequence. The VH and VL sequences were derived using SLAM (selected lymphocyte antibody method).

The primers were provided by Sigma Aldrich and Eurogentec.

For PCR1 of F2 VH the following primers were provided:

### P1:

A first primer (P1), as shown in SEQ ID NO: 3, comprising a region complementary to the 5' end of a variable heavy chain domain containing sequence (F2), specifically to a leader sequence at the 5' end of the variable heavy chain domain sequence, and an overlap-extension tail complementary to the 3' non-coding end of a promoter sequence (F1). SEQ ID NO: 4 is the amino acids sequence of the 5' end of the leader sequence of the variable heavy chain domain sequence (F2) encoded by SEQ ID NO: 3 of the first primer (P1).

### P2:

A second primer (P2), as show in SEQ ID NO: 1, comprising a region complementary to the 3' end of a variable heavy chain domain sequence (F2) and an overlap-extension tail complementary to the 5' end of a constant heavy chain domain and polyA sequence (F3). SEQ ID NO: 2 is the amino acid sequence encoded by SEQ ID NO: 1 of the second primer (P2), wherein QGTLVTVSS is the 3' end of the variable heavy chain domain (F2) and ASTKGP is the 5' end of the constant heavy chain domain and poly A sequence (F3). For PCR1 of F2 VL the following primers were provided:

### P1:

A first primer (P1), as shown in SEQ ID NO: 7, comprising a region complementary to the 5' end of a variable light chain domain containing sequence (F2), specifically to a leader sequence at the 5' end of the variable light chain domain sequence, and an overlap-extension tail complementary to the 3' non-coding end of a promoter sequence (F1). SEQ ID NO: 8 is the amino acids sequence of the 5' end of the leader sequence of the variable light chain domain sequence (F2) encoded by SEQ ID NO: 7 of the first primer (P1).

### P2:

A second primer (P2), as shown in SEQ ID NO: 5, comprising a region complementary to the 3' end of a variable light chain domain sequence and an overlap-extension tail complementary to the 5' end of a constant light chain domain and polyA sequence (F3). SEQ ID NO: 6 is the amino acid sequence encoded by SEQ ID NO: 5 of the second primer, wherein GTKVEIKR is the 3' end of the variable light chain domain and TVAAPSVF is the 5' end of the constant light chain domain and poly A sequence (F3).

### PCR1 Amplification of Variable Light Chain Sequence (F2 VL)

The F2 VL encoding sequence was amplified by PCR in the presence of primer P1 (SEQ ID NO: 7) and primer P2 (SEQ ID NO: 5), as set out above.

The VL encoding sequence was generated by restriction digest from an expression vector.

The reaction conditions used are provided below:

| | |
|---|---|
| 10 x KOD Buffer | 2.5µl |
| KOD polymerase | 0.5µl |
| dNTPs | 2.5µl |
| Primer (P1) (SEQ ID NO: 7) | 2.0µl (1.25pmol/µl) |
| Primer (P2) (SEQ ID NO: 5) | 2.0µl (1.25pmol/µl) |
| MgSO4 | 1.0µl |
| Water | 13.5µl |
| Template DNA (F2 VL) | 1.0µl * |

The PCR program KOD#R for PCR1 was carried out as follows:

| | |
|---|---|
| Step 1. 95°C | 2mins |
| Step 2. 95°C | 20secs |
| Step 3. 55°C | 10secs |
| Step 4. 72°C | 5secs and go to step 2 (steps 2 to 4 repeated 24 further times) |
| Step 5. 4°C | hold |

PCR1 generated an intermediate PCR product comprising the F2 VL sequence (approximately 420 bp) having a 5' end sequence complementary to the 3' end of the promoter sequence (F1) and a 3' end sequence complementary to the 5' end of the constant domain and polyadenylation sequence (F3). An agarose gel electrophoresis was carried out to confirm the production of the intermediate PCR product, as shown in Figure 3a the PCR1 VL is the intermediate PCR product for VL. In Figure 3a M1 is a standard DNA ladder from Bioline called Hyperladder 1, B is a blank negative control and 659 is the VL intermediate PCR product.

### PCR1 Amplification of Variable Heavy Chain Sequence (F2 VH)

As for PCR 1 of F2 VL, the F2 VH encoding sequence was amplified by PCR in the presence of primer P1 (SEQ ID NO: 3) and primer P2 (SEQ ID NO: 1), as set out above.

The reaction conditions used are provided below:

| | |
|---|---|
| 10 x KOD Buffer | 2.5µl |
| KOD polymerase | 0.5µl |
| dNTPs | 2.5µl |
| Primer (P1) (SEQ ID NO: 3) | 2.0µl (1.25pmol/µl) |
| Primer (P2) (SEQ ID NO: 1) | 2.0µl (1.25pmol/µl) |
| MgSO4 | 1.0µl |
| Water | 13.5µl |
| Template DNA (F2 VH) | 1.0µl * |

The VH sequence was generated by restriction digest from an expression vector.

The same PCR program cycles as set out above for PCR1 of F2 VL were also used for PCR1 of F2 VH.

PCR1 generated an intermediate PCR product comprising the F2 VH sequence (approximately 400bp) having a 5' end sequence complementary to the 3' end of the promoter sequence (F1) and a 3' end sequence complementary to the 5' end of the constant domain and polyadenylation sequence (F3). An agarose gel electrophoresis was carried out to confirm the production of the intermediate PCR product, as shown in Figure 4a the PCR1 VH is the intermediate PCR product for VH. In Figure 4a M2 is a standard DNA ladder from Novagen called Perfect™ 0.1-12kb, B is a blank negative control and 659 is the VH intermediate PCR product.

### Example 2 - The second PCR step (PCR2) in the generation of linear transcriptionally active polynucleotides.

### Primers for PCR 2

Primers were designed suitable for a second PCR amplification (PCR2) of the F2 VL intermediate PCR product and F2 VH intermediate PCR product from PCR1

For PCR2 of the intermediate PCR products of F2 VH and F2 VL the following primers were provided:

### P3:

A third primer (P3), as shown in SEQ ID NO: 10, is complementary to a non-coding region at the 5' end of a promoter sequence (F1).

### P4:

A fourth primer (P4), as shown in SEQ ID NO: 9, is complementary to a non-coding region at the 3' end of the polyadenylation sequence of F3.

### Generation of Constant Light Chain Domain and Poly A Sequence (F3)

A polynucleotide sequence comprising a murine constant light chain (Kappa) encoding sequence and a poly A sequence (F3) was generated by restriction digest from an expression vector.

The reaction conditions used are as follows:

| | |
|---|---|
| DNA template | 10µl (1ug/ul) |
| Buffer H(x10 Roche) | 5.0µl |
| BsiWI | 2µl (10U/µl from New England Biolabs) |
| NotI | 2µl (10U/µl from New England Biolabs) |
| Water | 31µl |

Reaction incubated at 37°C overnight (∼16 hours) to release a fragment of 598 base pairs comprising a murine constant light chain encoding sequence and a poly A sequence (F3).

The fragment was gel extracted and checked by agarose gel electorphoresis.

SEQ ID NO: 12 shows the nucleotide sequence comprising the constant mouse Kappa chain sequence and the polyA sequence. In SEQ ID NO: 12 the sequence of nucleotides 2 to 321 is the constant mouse Kappa chain sequence and the sequence of nucleotides 348 to 597 is the polyA sequence. Nucleotide 1 and nucleotides 598 to 605 are nucleotides from restriction sites for BsiWI and NotI due to generation of the sequence by restriction digest from a vector.

### Generation of Constant Heavy Chain Domain and Poly A Sequence (F3)

The murine constant full length murin gamma 1 isotype domain fragment with downstream polyA sequecne was generated by restriction digest from an expression vector.

The reaction conditions used are as follows.

| | |
|---|---|
| DNA template | 10µl (1ug/ul) |
| Buffer H(x10 Roche) | 5.0µl |
| XhoI | 2µl (10U/µl from New England Biolabs) |
| SalI | 2µl (10U/µl from New England Biolabs) |
| Water | 31µl |

Reaction incubated at 37°C overnight (∼16 hours) to release a fragment of 1578 base pairs. The fragment was gel extracted and checked by agarose gel electorphoresis.

SEQ ID NO: 13 shows the nucleotide sequence comprising the constant mouse gamma 1 chain sequence and the polyA sequence. In SEQ ID NO:13 the sequence of nucleotides 2 to 986 is the constant mouse gamma 1 chain sequence and the sequence of nucleotides 1059 to 1307 is the poly A sequence. Nucleotide 1 and nucleotides 1580 to 1585 are nucleotides from restriction sites for XhoI and SalI due to generation of the sequence by restriction digest from a vector.

### Generation of Promoter Sequence (F1):

A similar digest using the restriction enzymes MluI and HindIII was used to liberate the upstream hCMV promoter containing fragment (F1) of 2089 base pairs, as shown in SEQ ID NO: 11. In SEQ ID NO:11 the sequence of nucleotides 89 to 2080 is the hCMV promoter sequence. Nucleotides 1 to 6 and nucleotides 2084 to 2089 are nucleotides from restriction sites for MluI and HindIII due to generation of the sequence by restriction digest from a vector.

### PCR 2 Amplification of the Intermediate PCR Product of Variable Light Chain Sequence (F2 VL)

The intermediate PCR product of F2 VL was amplified by PCR in the presence of primer P3 (SEQ ID NO: 10) and primer P4 (SEQ ID NO: 9), a promoter sequence F1 (SEQ ID NO: 11) and a sequence comprising the constant light chain sequence and poly A sequence F3 (SEQ ID NO: 12).

The reaction conditions used are as follows:

| | |
|---|---|
| 10 x KOD Buffer | 2.5µl |
| KOD polymerase | 0.5µl |
| dNTPs | 2.5µl |
| Primer 3 (P3 SEQ ID NO: 10) | 2.0µl (1.25pmol/µl) |
| Primer 4 (P4 SEQ ID NO: 9) | 2.0µl (1.25pmol/µl) |
| Constant Light & polyA fragment (F3 SEQ ID NO : 12) | 1.0µl (2ng/µl) |
| Promoter Sequence (F1 SEQ ID NO: 11) | 1.0µl (2ng/µl) |
| MgSO4 | 1.0µl |
| Water | 10.5µl |
| Template DNA (intermediate PCR product F2 VL) | 2.0µl |

The reaction conditions used for PCR2 are provided below:
The PCR program KOD#T for PT-PCR is...

| | |
|---|---|
| Step 1. 95°C | 2mins |
| Step 2. 95°C | 20secs |
| Step 3. 55°C | 30secs |
| Step 4. 72°C | 2 mins and go to Step 2 (steps 2 to 4 repeated 29 further times) |
| Step 5. 4°C | hold |

PCR2 generated the final linear transcriptionally active PCR product (TAP light) comprising the promoter sequence (F1) (approx 2100bp), the F2 VL sequence (approximately 420 bp) and the constant domain and polyadenylation sequence F3 (approximately 600bp). An agarose gel electrophoresis was carried out to confirm the production of the final PCR product, as shown in Figure 3b the PCR2 VL is the final linear transcriptionally active polynucleotide for VL, which has the expected size. In Figure 3b M2 is a standard DNA ladder from Novagen called Perfect™ 0.1-12kb, B is a blank negative control and 659 is the final PCR product

### PCR 2 Amplification of the Intermediate PCR Product of Variable Heavy Chain Sequence (F2 VH)

As for PCR 2 of the intermediate PCR product of F2 VL, the intermediate PCR product of F2 VH was amplified by PCR in the presence of primer P3 (SEQ ID NO: 10) and primer P4 (SEQ ID NO: 9), a promoter sequence F1 (SEQ ID NO: 11) and a sequence comprising the constant heavy chain sequence and poly A sequence F3 (SEQ ID NO: 13).

The reaction conditions used are provided below:

| | |
|---|---|
| 10 x KOD Buffer | 2.5µl |
| KOD polymerase | 0.5µl |
| dNTPs | 2.5µl |
| Primer 3 (P3 SEQ ID NO: 10) | 2.0µl (1.25pmol/µl) |
| Primer 4 (P4 SEQ ID NO: 9) | 2.0µl (1.25pmol/µl) |
| Constant Heavy & polyA fragment (F3 SEQ ID NO : 13) | 1.0µl (2ng/µl) |
| Promoter Sequence (F1 SEQ ID NO: 11) | 1.0µl (2ng/µl) |
| MgSO4 | 1.0µl |
| Water | 10.5µl |
| Template DNA(intermediate PCR product F2 VH) | 2.0µl |

The same PCR program cycles as set out above for PCR2 of the intermediate PCR product of F2 VL were also used for PCR2 of the intermediate PCR product of F2 VH.

PCR2 generated the final linear transcriptionally active PCR product (TAP heavy) comprising the promoter sequence (F1) (approx 2100bp), the F2 VH sequence (approximately 400bp) and the constant domain and polyadenylation sequence F3 (approximately 1600bp). An agarose gel electrophoresis was carried out to confirm the production of the final PCR product, as shown in Figure 4b the PCR2 VH is the final linear transcriptionally active polynucleotide for VH, which has the expected size. In Figure 4b M2 is a standard DNA ladder from Novagen called Perfect™ 0.1-12kb, B is a blank negative control and 659 is the final PCR product

### Example 3 - Transfection of the TAP heavy and TAP light into host cells.

6-well plates (10 cm², 35mm per well) were used to carry out transfection of the TAP heavy and TAP light products of PCR 2 in Example 2. Approximately 5µg of TAP DNA was transferred into each well comprising 2.5µg of TAP heavy and 2.5µg of TAP light. This is approximately 5 µl of each PT-PCR giving 10µl which was added to 170µl of Opti-MEM medium in polycarbonate microfuge tubes.

Another 170µl Opti-MEM is added to 10µl of 293fectin in a new polycarbonate tube for each well of cells to be transfected. This mix was incubated for 5mins at room temperature.

The DNA Opti-MEM mix and the 293fectin Opti-MEM mixes were then combined and incubated at room temperature for 20mins.

HEK293 cells were prepared in FreeStyle™ media, at a cell density of 1x10⁶/ml with 5ml of cells per transfection well. Cells were dispensed into the individual wells of the 6 well plate and the Opti-MEM mix added directly to the well.

The plate was then incubated on a gently shaking platform in a humidified CO₂ incubator for 4 to 5 days.

The antibody was expressed and secreted into the supernatant at an approximate level of 2µg/ml.

Similar protocols for expression of antibodies using the linear transcriptionally active polynucleotides has provided yields of up 9µg/ml of antibody.

### Example 4 - Analysis of Expressed Antibody Activity using ELISA

The full length murine IgG antibody produced from the transient transfection of host cells in Example 3 was analyzed for activity using a standard antigen based ELISA assay, with the following protocol:
Each well of a 96 well Nunc Maxisorp™ bioassay plate was coated with 100µl of anti-mouse IgG Fcgamma specific F(ab')₂ antibody supplied by Jackson (Catalogue Number 115-006-071) at 1µg/ml in phosphate buffered saline.

The plate was washed three times with 300µl PBS 0.1%Tween 20 in an automated plate washer.

Each well of the plate was the blocked with 200µl a 1%(weight/volume) solution of Bovine Serum Albumin (BSA) in PBS and incubated at room temperature for at least one hour.

A serial dilution of a mouse gamma 1 antibody "MOPC21" of known concentration was used as a standard for this assay.

This standard and dilutions of the samples from the supernatants from Example 3 containing the TAP heavy and TAP light produced murine IgG antibody were subsequently detected using 100µl of goat anti-mouse IgG F(ab')₂ peroxidase conjugate, supplied by Jackson (Catalogue Number 115-035-072) diluted 1/5000 in blocking solution, incubated for 1hour.

The ELISA was developed by the addition of 100µl TMB (3-3',5,5'-tetramethylbenzidine) reagent and read at 630nM using an automated spectrophotometic plate reader.

The antibody was measured at 0.45ug/ml

This sample was analyzed by the Biacore surface Plasmon resonance.

A sample comprising the murine IgG antibody expressed in Example 3 was captured onto a carboxymethylated dextran micro-chip surface to which an anti-mouse antibody had been immobilised irreversibly.

The immobilised anti-mouse antibody stably captured the murine IgG antibody expressed in Example 3. Then the antigen of murine IgG antibody was passed over at known concentrations and the resulting data produced by the increase in mass (measured by a technique called surface plasmon resonance) allows determination of the affinity between the two binding partners, murine IgG antibody and the antigen. An affinity value of 0.367pM was measured.

The results from the ELISA assay and the Biocore assay show conclusively that the antibody was being expressed and retained antigen specificity with predicted affinity compared to previously expressed samples of the antibody produced by standard cloning methods.

While this invention has been particularly shown and described with reference to the preferred embodiments, it will be understood to those skilled in the art that various changes in form and detail may be made without departing from the scope of the invention as defined by the appendant claims.

## Claims

1. A method for obtaining a recombinant antibody with a desired ability to bind a selected antigen which avoids multiple digestion and ligation steps to produce vectors and multiple transformations into host cells, comprising:
(a) providing a population of antibody-forming lymphocyte cells suspected of containing at least one cell capable of producing an antibody comprising a heavy chain and a light chain exhibiting said desired specificity;
(aa) a first screening step prior to step (b) in which the population of antibody-forming cells provided in step (a) are screened to identify an antibody-forming cell or a population of antibody-forming cells producing an antibody exhibiting the desired ability;
(aaa) identifying and isolating a single antibody-forming cell producing an antibody exhibiting the desired ability from a population of antibody-forming cells identified by the first screening step (aa);
(b) generating a transcriptionally active recombinant linear polynucleotide encoding a light chain or a fragment thereof and a transcriptionally active recombinant linear polynucleotide encoding a heavy chain or a fragment thereof for each recombinant antibody from the single isolated antibody forming cell obtained in step (aaa), wherein each transcriptionally active recombinant linear polynucleotide comprises a first encoding polynucleotide sequence encoding a variable domain of an antibody produced by the single isolated antibody-forming cell obtained in step (aaa), a second encoding polynucleotide sequence encoding one or more constant domain encoding polynucleotide sequences and one or more transcription regulatory elements and is not capable of autonomous replication; wherein step (b) comprises:
bb) providing the first encoding polynucleotide sequence, the second encoding polynucleotide sequence and the one or more transcription regulatory elements;
bbb) fusing the first encoding polynucleotide sequence and the second encoding polynucleotide sequence by PCR, and
bbbb) fusing one or more transcription regulatory elements to the first encoding polynucleotide sequence and/or the second encoding polynucleotide sequence by PCR;
(c) expressing a recombinant antibody comprising a heavy chain and a light chain from the transcriptionally active recombinant linear polynucleotides generated in step (b) by transiently transfecting the transcriptionally active recombinant linear polynucleotides into a cell, growing said host cells in an appropriate medium and isolating the antibody from the cell;
(d) screening the recombinant antibody produced by step (c) for the desired ability; and
(e) optionally repeating steps (b), (c) and (d) to identify a recombinant antibody exhibiting the desired ability.

2. The method according to claim 1, wherein the recombinant antibody expressed in step (c) retains the original light chain and heavy chain pairing of an antibody produced by an antibody-forming cell.

3. The method according to any preceding claim, wherein the light chain or a fragment thereof is encoded by a variable domain encoding polynucleotide sequence and a light chain constant domain encoding polynucleotide sequence and the heavy chain or a fragment thereof is encoded by a variable domain encoding polynucleotide sequence and CH1, CH2 and CH3 constant domain encoding polynucleotide sequences.

4. The method according to any preceding claim, wherein the antibody-forming cells are obtained from an animal which has been immunized with a selected antigen.

5. The method according to any of claims 1 to 3, wherein the antibody-forming cells are obtained from an animal which generated said cells during the course of a selected disease.

6. The method according to any preceding claim, wherein the transcriptionally active recombinant linear polynucleotides do not comprise an origin of replication.

7. The method according to any preceding claim, wherein each transcriptionally active recombinant linear polynucleotide comprises a first transcription regulatory element comprising a promoter and a second transcription regulatory sequence capable of terminating transcription and/or initiating cleavage of the RNA transcript.

8. The method according to any preceding claim, wherein the first screening step aa) and the isolating step aaa) are carried out simultaneously, step (aa) comprises suspending the population of antibody-forming cells provided in step (a) in a medium, the medium having an indicator system incorporated therein, said indicator system being capable of indicating the presence and location of an antibody-forming cell or a population of antibody-forming cells producing an antibody exhibiting the desired ability and step (aaa) comprises identifying and isolating the single antibody-forming cell.

9. The method according to claim 8, wherein the indicator system comprises a selected antigen and a labeled anti-antibody antibody.

## Patentansprüche

1. Verfahren zum Erhalt eines rekombinanten Antikörpers mit einer gewünschten Fähigkeit zur Bindung eines ausgewählten Antigens, bei dem mehrfache Verdauungs- und Ligationsschritte zum Herstellen von Vektoren und mehrfache Transformationen in Wirtszellen vermieden werden, umfassend:
(a) Bereitstellen einer Population antikörperbildender Lymphozyten-Zellen, von der angenommen wird, dass sie wenigstens eine Zelle enthält, die einen eine schwere Kette und eine leichte Kette, die die gewünschte Spezifität zeigen, umfassenden Antikörper produzieren kann;
(aa) einen ersten Screening-Schritt vor Schritt (b), bei dem die in Schritt (a) bereitgestellte Population antikörperbildender Zellen einem Screening unterzogen wird, um eine antikörperbildende Zelle oder eine Population antikörperbildender Zellen, die einen die gewünschte Fähigkeit zeigenden Antikörper produziert, zu identifizieren,;
(aaa) Identifizieren und Isolieren einer antikörperbildenden Einzelzelle, die einen die gewünschte Fähigkeit zeigenden Antikörper produziert, aus einer Population antikörperbildender Zellen, die durch den ersten Screening-Schritt (aa) identifiziert wurden;
(b) Erzeugen eines transkriptionell aktiven rekombinanten linearen Polynukleotids, das eine leichte Kette oder ein Fragment davon codiert, und eines transkriptionell aktiven rekombinanten linearen Polynukleotids, das eine schwere Kette oder ein Fragment davon codiert, für jeden rekombinanten Antikörper aus der in Schritt (aaa) erhaltenen isolierten antikörperbildenden Einzelzelle, wobei jedes transkriptionell aktive rekombinante lineare Polynukleotid eine erste codierende Polynukleotidsequenz, die eine variable Domäne eines von der in Schritt (aaa) erhaltenen isolierten antikörperbildenden Einzelzelle produzierten Antikörpers codiert, eine zweite codierende Polynukleotidsequenz, die eine oder mehrere konstante-Domäne-codierende Polynukleotidsequenzen codiert, und ein oder mehrere transkriptionsregulatorische Elemente umfasst und nicht zur autonomen Replikation fähig ist; wobei Schritt (b) Folgendes umfasst:
(bb) Bereitstellen der ersten codierenden Polynukleotidsequenz, der zweiten codierenden Polynukleotidsequenz und des einen bzw. der mehreren transkriptionsregulatorischen Elements bzw. Elemente;
bbb) Fusionieren der ersten codierenden Polynukleotidsequenz und der zweiten codierenden Polynukleotidsequenz durch PCR und
bbbb) Fusionieren eines oder mehrerer transkriptionsregulatorischer Elemente an die erste codierende Polynukleotidsequenz und/oder die zweite codierende Polynukleotidsequenz durch PCR;
(c) Exprimieren eines eine schwere Kette und eine leichte Kette umfassenden rekombinanten Antikörpers aus den in Schritt (b) erzeugten transkriptionell aktiven rekombinanten linearen Polynukleotiden, indem die transkriptionell aktiven rekombinanten linearen Polynukleotide in eine Zelle transient transfiziert werden, die Wirtszellen in einem entsprechenden Medium kultiviert werden und der Antikörper aus der Zelle isoliert wird;
(d) Screening des durch Schritt (c) produzierten rekombinanten Antikörpers auf die gewünschte Fähigkeit; und
(e) gegebenenfalls Wiederholen der Schritte (b), (c) und (d) zum Identifizieren eines rekombinanten Antikörpers, der die gewünschte Fähigkeit zeigt.

2. Verfahren nach Anspruch 1, wobei der in Schritt (c) exprimierte rekombinante Antikörper die ursprüngliche Paarung von leichter Kette und schwerer Kette eines von einer antikörperbildenden Zelle produzierten Antikörpers beibehält.

3. Verfahren nach einem vorhergehenden Anspruch, wobei die leichte Kette oder ein Fragment davon durch eine variable-Domäne-codierende Polynukleotidsequenz und eine leichte-Kette-konstante-Domäne-codierende Polynukleotidsequenz codiert ist und die schwere Kette oder ein Fragment davon durch eine variable-Domäne-codierende Polynukleotidsequenz und CH1-, CH2- und CH3-konstante-Domäne-codierende Polynukleotidsequenzen codiert ist.

4. Verfahren nach einem vorhergehenden Anspruch, wobei die antikörperbildenden Zellen einem Tier entnommen werden, das mit einem ausgewählten Antigen immunisiert wurde.

5. Verfahren nach einem der Ansprüche 1 bis 3, wobei die antikörperbildenden Zellen einem Tier entnommen werden, das die Zellen im Verlauf einer ausgewählten Krankheit erzeugte.

6. Verfahren nach einem vorhergehenden Anspruch, wobei die transkriptionell aktiven rekombinanten linearen Polynukleotide keinen Replikationsursprung umfassen.

7. Verfahren nach einem vorhergehenden Anspruch, wobei jedes transkriptionell aktive rekombinante lineare Polynukleotid ein erstes transkriptionsregulatorisches Element, das einen Promotor umfasst, und eine zweite transkriptionsregulatorische Sequenz, das zur Transkriptionstermination und/oder zum Initiieren der Spaltung des RNA-Transkripts fähig ist, umfasst.

8. Verfahren nach einem vorhergehenden Anspruch, wobei der erste Screening-Schritt aa) und der Isolierungsschritt aaa) gleichzeitig durchgeführt werden, Schritt (aa) Suspendieren der in Schritt (a) bereitgestellten Population antikörperbildender Zellen in einem Medium umfasst, wobei das Medium ein darin eingebautes Indikatorsystem aufweist, wobei das Indikatorsystem das Vorliegen und den Ort einer antikörperbildenden Zelle oder einer Population antikörperbildender Zellen, die einen die gewünschte Fähigkeit zeigenden Antikörper produziert, anzeigen kann, und Schritt (aaa) Identifizieren und Isolieren der antikörperbildenden Einzelzelle umfasst.

9. Verfahren nach Anspruch 8, wobei das Indikatorsystem ein ausgewähltes Antigen und einen markierten Anti-Antikörper-Antikörper umfasst.

## Revendications

1. Procédé d'obtention d'un anticorps recombinant ayant une capacité souhaitée de liaison à un antigène sélectionné qui évite des étapes de digestion et ligature multiples pour produire des vecteurs et des transformations multiples dans des cellules hôtes, comprenant :
(a) la fourniture d'une population de cellules lymphocytaires formant des anticorps suspectée de contenir au moins une cellule capable de produire un anticorps comprenant une chaîne lourde et une chaîne légère présentant ladite spécificité souhaitée ;
(aa) une première étape de criblage avant l'étape (b) dans laquelle la population de cellules formant des anticorps fournie dans l'étape (a) est criblée pour identifier une cellule formant des anticorps ou une population de cellules formant des anticorps produisant un anticorps présentant la capacité souhaitée ;
(aaa) l'identification et l'isolement d'une cellule formant des anticorps individuelle produisant un anticorps présentant la capacité souhaitée à partir d'une population de cellules formant des anticorps identifiées par la première étape de criblage (aa) ;
(b) la génération d'un polynucléotide linéaire recombinant transcriptionnellement actif codant pour une chaîne légère ou un fragment de celle-ci et d'un polynucléotide linéaire recombinant transcriptionnellement actif codant pour une chaîne lourde ou un fragment de celle-ci pour chaque anticorps recombinant à partir de la cellule formant des anticorps isolée individuelle obtenue dans l'étape (aaa), dans lequel chaque polynucléotide linéaire recombinant transcriptionnellement actif comprend une première séquence de polynucléotide codante codant pour un domaine variable d'un anticorps produit par la cellule formant des anticorps isolée individuelle obtenue dans l'étape (aaa), une deuxième séquence de polynucléotide codante codant pour une ou plusieurs séquences de polynucléotide codant pour un ou plusieurs domaines constants et un ou plusieurs éléments régulateurs de transcription et n'est pas capable de réplication autonome ; dans lequel l'étape (b) comprend :
bb) la fourniture de la première séquence de polynucléotide codante, la deuxième séquence de polynucléotide codante et les un ou plusieurs éléments régulateurs de transcription ;
bbb) la fusion de la première séquence de polynucléotide codante et la deuxième séquence de polynucléotide codante par PCR, et
bbbb) la fusion d'un ou plusieurs éléments régulateurs de transcription à la première séquence de polynucléotide codante et/ou la deuxième séquence de polynucléotide codante par PCR ;
(c) l'expression d'un anticorps recombinant comprenant une chaîne lourde et une chaîne légère à partir des polynucléotides linéaires recombinants transcriptionnellement actifs générés dans l'étape (b) par transfection transitoire des polynucléotides linéaires recombinants transcriptionnellement actifs dans une cellule, la culture desdites cellules hôtes dans un milieu approprié et l'isolement de l'anticorps à partir de la cellule ;
(d) le criblage de l'anticorps recombinant produit dans l'étape (c) pour la capacité souhaitée ; et
(e) facultativement, la répétition des étapes (b), (c) et (d) pour identifier un anticorps recombinant présentant la capacité souhaitée.

2. Procédé selon la revendication 1, dans lequel l'anticorps recombinant exprimé dans l'étape (c) conserve l'appariement original de chaîne légère et de chaîne lourde d'un anticorps produit par une cellule formant des anticorps.

3. Procédé selon l'une quelconque des revendications précédentes, dans lequel la chaîne légère ou un fragment de celle-ci est codée par une séquence de polynucléotide codant pour un domaine variable et une séquence de polynucléotide codant pour un domaine constant de chaîne légère et la chaîne lourde ou un fragment de celle-ci est codée par une séquence de polynucléotide codant pour un domaine variable et des séquences de polynucléotide codant pour des domaines constants CH1, CH2 et CH3.

4. Procédé selon l'une quelconque des revendications précédentes, dans lequel les cellules formant des anticorps sont obtenues à partir d'un animal qui a été immunisé avec un antigène sélectionné.

5. Procédé selon l'une quelconque des revendications 1 à 3, dans lequel les cellules formant des anticorps sont obtenues à partir d'un animal qui a généré lesdites cellules au cours d'une maladie sélectionnée.

6. Procédé selon l'une quelconque des revendications précédentes, dans lequel les polynucléotides linéaires recombinants transcriptionnellement actifs ne comprennent pas une origine de réplication.

7. Procédé selon l'une quelconque des revendications précédentes, dans lequel chaque polynucléotide linéaire recombinant transcriptionnellement actif comprend un premier élément régulateur de transcription comprenant un promoteur et une deuxième séquence régulatrice de transcription capable de terminer la transcription et/ou d'initier le clivage du produit de transcription ARN.

8. Procédé selon l'une quelconque des revendications précédentes, dans lequel la première étape de criblage aa) et l'étape d'isolement aaa) sont conduites simultanément, l'étape (aa) comprend la mise en suspension de la population de cellules formant des anticorps fournie dans l'étape (a) dans un milieu, le milieu comportant un système d'indicateur incorporé dans celui-ci, ledit système d'indicateur étant capable d'indiquer la présence et l'emplacement d'un cellule formant des anticorps ou d'une population de cellules formant des anticorps produisant un anticorps présentant la capacité souhaitée et l'étape (aaa) comprend l'identification et l'isolement de la cellule individuelle formant des anticorps.

9. Procédé selon la revendication 8, dans lequel le système d'indicateur comprend un antigène sélectionné et un anticorps anti-anticorps marqué.


### SEQUENCE LISTING

<110> UCB Pharma S.A.
<120> Method for producing proteins
<130> G0081-WO01
<150> GB0903209.5
   <151> 2009-02-25
<150> GB0903210.3
   <151> 2009-02-25
<160> 13
<170> PatentIn version 3.5
<210> 1
   <211> 49
   <212> DNA
   <213> Artificial Sequence
<220>
   <223> Primer Sequence
<400> 1
   gccagggaac tcttgtgaca gtctcgagcg cttctacaaa gggcccatc 49
<210> 2
   <211> 15
   <212> PRT
   <213> Artificial Sequence
<220>
   <223> Encoded sequence of primer
<400> 2
   Gln Gly Thr Leu Val Thr Val Ser Ser Ala Ser Thr Lys Gly Pro
   1               5                   10                  15
<210> 3
   <211> 47
   <212> DNA
   <213> Artificial Sequence
<220>
   <223> Primer Sequence
<400> 3
   gcagtcaccg tccttgacac gaagcttgcc accatggagt ggtcctg 47
<210> 4
   <211> 4
   <212> PRT
   <213> Artificial Sequence
<220>
   <223> Sequence encoded by primer
<400> 4
   Met Glu Trp Ser
1
<210> 5
   <211> 49
   <212> DNA
   <213> Artificial Sequence
<220>
   <223> Primer Sequence
<400> 5
   gaagacagat ggggccgcta ccgtacgctt gatctccact ttagtgccc 49
<210> 6
   <211> 16
   <212> PRT
   <213> Artificial Sequence
<220>
   <223> Sequence encoded by primer
<400> 6
   Gly Thr Lys Val Glu Ile Lys Arg Thr Val Ala Ala Pro Ser Val Phe
   1 5 10 15
<210> 7
   <211> 50
   <212> DNA
   <213> Artificial Sequence
<220>
   <223> Primer Sequence
<400> 7
   cgtccttgac acgaagctat tcgtaagctt gccaccatgt ccgttcccac 50
<210> 8
   <211> 4
   <212> PRT
   <213> Artificial Sequence
<220>
   <223> Sequence encoded by primer
<400> 8
   Met Ser Val Pro
   1
<210> 9
   <211> 28
   <212> DNA
   <213> Artificial Sequence
<220>
   <223> Primer sequence
<400> 9
   acatgataag atacattgat gagtttgg 28
<210> 10
   <211> 42
   <212> DNA
   <213> Artificial Sequence
<220>
   <223> Primer sequence
<400> 10
   acgcgttttg agatttctgt cgccgactaa attcatgtcg cg 42
<210> 11
   <211> 2089
   <212> DNA
   <213> Artificial Sequence
<220>
   <223> Sequence comprising hCMV promoter sequence
<400> 11
   acgcgttttg agatttctgt cgccgactaa attcatgtcg cgcgatagtg gtgtttatcg 60
   ccgatagaga tggcgatatt ggaaaaatcg atatttgaaa atatggcata ttgaaaatgt 120
   cgccgatgtg agtttctgtg taactgatat cgccattttt ccaaaagtga tttttgggca 180
   tacgcgatat ctggcgatag cgcttatatc gtttacgggg gatggcgata gacgactttg 240
   gtgacttggg cgattctgtg tgtcgcaaat atcgcagttt cgatataggt gacagacgat 300
   atgaggctat atcgccgata gaggcgacat caagctggca catggccaat gcatatcgat 360
   ctatacattg aatcaatatt ggccattagc catattattc attggttata tagcataaat 420
   caatattggc tattggccat tgcatacgtt gtatccatat cataatatgt acatttatat 480
   tggctcatgt ccaacattac cgccatgttg acattgatta ttgactagtt attaatagta 540
   atcaattacg gggtcattag ttcatagccc atatatggag ttccgcgtta cataacttac 600
   ggtaaatggc ccgcctggct gaccgcccaa cgacccccgc ccattgacgt caataatgac 660
   gtatgttccc atagtaacgc caatagggac tttccattga cgtcaatggg tggagtattt 720
   acggtaaact gcccacttgg cagtacatca agtgtatcat atgccaagta cgccccctat 780
   tgacgtcaat gacggtaaat ggcccgcctg gcattatgcc cagtacatga ccttatggga     840
   ctttcctact tggcagtaca tctacgtatt agtcatcgct attaccatgg tgatgcggtt     900
   ttggcagtac atcaatgggc gtggatagcg gtttgactca cggggatttc caagtctcca     960
   ccccattgac gtcaatggga gtttgttttg gcaccaaaat caacgggact ttccaaaatg    1020
   tcgtaacaac tccgccccat tgacgcaaat gggcggtagg cgtgtacggt gggaggtcta    1080
   tataagcaga gctcgtttag tgaaccgtca gatcgcctgg agacgccatc cacgctgttt    1140
   tgacctccat agaagacacc gggaccgatc cagcctccgc ggccgggaac ggtgcattgg    1200
   aacgcggatt ccccgtgcca agagtgacgt aagtaccgcc tatagagtct ataggcccac    1260
   ccccttggct tcttatgcat gctatactgt ttttggcttg gggtctatac acccccgctt    1320
   cctcatgtta taggtgatgg tatagcttag cctataggtg tgggttattg accattattg    1380
   accactcccc tattggtgac gatactttcc attactaatc cataacatgg ctctttgcca    1440
   caactctctt tattggctat atgccaatac actgtccttc agagactgac acggactctg    1500
   tatttttaca ggatggggtc tcatttatta tttacaaatt cacatataca acaccaccgt    1560
   ccccagtgcc cgcagttttt attaaacata acgtgggatc tccacgcgaa tctcgggtac    1620
   gtgttccgga catgggctct tctccggtag cggcggagct tctacatccg agccctgctc    1680
   ccatgcctcc agcgactcat ggtcgctcgg cagctccttg ctcctaacag tggaggccag    1740
   acttaggcac agcacgatgc ccaccaccac cagtgtgccg cacaaggccg tggcggtagg    1800
   gtatgtgtct gaaaatgagc tcggggagcg ggcttgcacc gctgacgcat ttggaagact    1860
   taaggcagcg gcagaagaag atgcaggcag ctgagttgtt gtgttctgat aagagtcaga    1920
   ggtaactccc gttgcggtgc tgttaacggt ggagggcagt gtagtctgag cagtactcgt    1980
   tgctgccgcg cgcgccacca gacataatag ctgacagact aacagactgt tcctttccat    2040
   gggtcttttc tgcagtcacc gtccttgaca cgaagctatt cgtaggcct                2089
<210> 12
   <211> 605
   <212> DNA
   <213> Artificial sequence
<220>
   <223> Sequence comprising mouse constant light chain sequence and poly A sequence
<400> 12
   cgtacggatg ctgcaccaac tgtatccatc ttcccaccat ccagtgagca gttaacatct     60
   ggaggtgcct cagtcgtgtg cttcttgaac aacttctacc ccaaagacat caatgtcaag    120
   tggaagattg atggcagtga acgacaaaat ggcgtcctga acagttggac tgatcaggac    180
   agcaaagaca gcacctacag catgagcagc accctcacgt tgaccaagga cgagtatgaa    240
   cgacataaca gctatacctg tgaggccact cacaagacat caacttcacc cattgtcaag    300
   agcttcaaca ggaatgagtg ttagctgctc ctcagttcca gcctgggaat tcattgatca    360
   ttaatcagcc ataccacatt tgtagaggtt ttacttgctt taaaaaacct cccacacctc    420
   cccctgaacc tgaaacataa aatgaatgca attgttgttg ttaacttgtt tattgcagct    480
   tataatggtt acaaataaag caatagcatc acaaatttca caaataaagc atttttttca    540
   ctgcattcta gttgtggttt gtccaaactc atcaatgtat cttatcatgt ctggatcgcg    600
   gccgc                                                                605
<210> 13
   <211> 1585
   <212> DNA
   <213> Artificial Sequence
<220>
   <223> Sequence comprising mouse gamma 1 constant heavy chain sequence and polyA sequence
<400> 13
   ctcgagtgcc aaaacgacac ccccatctgt ctatccactg gcccctggat ctgctgccca     60
   aactaactcc atggtgaccc tgggatgcct ggtcaagggc tatttccctg agccagtgac 120
   agtgacctgg aactctggat ccctgtccag cggtgtgcac accttcccag ctgtcctgca 180
   gtctgacctc tacactctga gcagctcagt gactgtcccc tccagcacct ggcccagcga 240
   gaccgtcacc tgcaacgttg cccacccggc cagcagcacc aaggtggaca agaaaattgt 300
   gcccagggat tgtggttgta agccttgcat atgtacagtc ccagaagtat catctgtctt 360
   catcttcccc ccaaagccca aggatgtgct caccattact ctgactccta aggtcacgtg 420
   tgttgtggta gacatcagca aggatgatcc cgaggtccag ttcagctggt ttgtagatga 480
   tgtggaggtg cacacagctc agacgcaacc ccgggaggag cagttcaaca gcactttccg 540
   ctcagtcagt gaacttccca tcatgcacca ggactggctc aatggcaagg agttcaaatg 600
   cagggtcaac agtgcagctt tccctgcccc catcgagaaa accatctcca aaaccaaagg 660
   cagaccgaag gctccacagg tgtacaccat tccacctccc aaggagcaga tggccaagga 720
   taaagtcagt ctgacctgca tgataacaga cttcttccct gaagacatta ctgtggagtg 780
   gcagtggaat gggcagccag cggagaacta caagaacact cagcccatca tggacacaga 840
   tggctcttac ttcgtctaca gcaagctcaa tgtgcagaag agcaactggg aggcaggaaa 900
   tactttcacc tgctctgtgt tacatgaggg cctgcacaac caccatactg agaagagcct 960
   ctcccactct cctggtaaat gatcccagtg tccttggagc cctctggtcc tacaggactc 1020
   tgacacctac ctccacccct ccctgtataa ataataagaa ttcattgatc ataatcagcc 1080
   ataccacatt tgtagaggtt ttacttgctt taaaaaacct cccacacctc cccctgaacc 1140
   tgaaacataa aatgaatgca attgttgttg ttaacttgtt tattgcagct tataatggtt 1200
   acaaataaag caatagcatc acaaatttca caaataaagc atttttttca ctgcattcta 1260
   gttgtggttt gtccaaactc atcaatgtat cttatcatgt ctggatcctc tacgccggac 1320
   gcatcgtggc cggcatcacc ggcgccacag gtgcggttgc tggcgcctat atcgccgaca 1380
   tcaccgatgg ggaagatcgg gctcgccact tcgggctcat gagcgcttgt ttcggcgtgg   1440
   gtatggtggc aggccccgtg gccgggggac tgttgggcgc catctccttg catgcaccat   1500
   tccttgcggc ggcggtgctc aacggcctca acctactact gggctgcttc ctaatgcagg   1560
   agtcgcataa gggagagcgt cgact                                         1585